# EUROPEAN PATENT APPLICATION

(11) **EP 4 261 231 A1**
(43) Date of publication of application: **18.10.2023**
(21) Application number: 21901953.6
(22) Date of filing: 19.05.2021
(51) Int. Cl.: C07K 19/00, A61K 39/395, A61P 37/02, A61P 31/00, A61P 35/00

(54) **BISPECIFIC ANTIBODY AND APPLICATION THEREOF**

(30) Priority: 09.12.2020 CN 202011427863
(71) Applicant: Qure Biotechnology (Shanghai) Co., Ltd., Shanghai 200120 (CN)
(72) Inventor: QU, Xiangdong, Shanghai 200120 (CN)
(74) Representative: Cabinet Laurent & Charras
(86) International application number: PCT/CN2021/094672
(87) International publication number: WO 2022/121239

(57) **Abstract**

The present invention provides a bispecific antibody and an application thereof. IL15 and IL15Rα are respectively introduced into two chains of a first antibody of the bispecific antibody of the present invention (for example, IL 15 and IL 15Rα replace CLl and CHI, respectively), and the high affinity of IL15 and IL15Rα is utilized to form an IL15/IL15Rα complex, thereby achieving the correct pairing of a light chain/heavy chain of the first antibody, and solving the problem of incorrectly matching light chains/heavy chains of bispecific antibodies. Meanwhile, the binding activity between the light chain/heavy chain of the first antibody may be further enhanced by adding one or more pairs of disulfide bonds between VH1 and VL1 and between IL15 and IL15Rαby mutating the amino acid sequences of variable domains VH1, VL1, IL15 and IL15Ra of the first antibody, to thereby effectively overcome challenges such as incorrectly matching light chains/heavy chains, high by-products and poor stability in the preparation process of a bispecific antibody, and finally prepare and obtain correctly paired bispecific multifunctional antibodies targeting cytokines. At the same time, the development period of bispecific antibodies is shortened, and production costs are reduced.

## Description

### TECHNICAL FIELD

The present invention belongs to the field of biomedicine, and relates to a bispecific antibody, and application thereof.

### BACKGROUND

Bispecific antibodies (BsAbs), also known as bifunctional antibodies, can recognize and bind two different antigens and epitopes at the same time, and block two different signaling pathways to play its role. Compared with ordinary antibodies, the BsAb incorporates a specific antigen binding site and shows the following advantages in terms of treatment:
Mediating immune cells to kill tumors: an important mechanism of bispecific antibodies is to mediate immune cell killing. A bispecific antibody has two antigen-binding arms, one of which binds to the target antigen and the other binds to the marker antigen on the effector cell, which can activate the effector cell to target and kill the tumor cell. The two bispecific antibody products currently approved for marketing belong to this category, with catumaxomab developed by Trion Pharma, which targets the tumor surface antigen EpCAM and the T cell surface receptor CD3, and the Blinatumomab developed by Micromet and Amgen which simultaneously binds to CD19 and CD3. Both of which serve the purpose of treating tumors by activating and recruiting killer T cells.

Blocking dual target signaling, playing a unique or overlapping function, and effectively preventing drug resistance: combining with dual targets and blocking the dual signal pathway at the same time is another important mechanism of bispecific antibodies. Receptor tyrosine kinases (RTKs) are the largest class of enzyme-linked receptors, which play an important role in the process of cell proliferation, such as Her family, etc. The abnormal high expression of RTKs on the surface of tumor cells leads to malignant proliferation of tumor cells, so they are also important targets for tumor therapy. Single-target monoclonal antibodies against RTKs have been widely used in cancer therapy, but tumor cells can perform immune escape by switching signaling pathways or by activating intracellular signals through homo- or heterodimers of HER family members themselves or between different members. Therefore, the use of bispecific antibody drugs to simultaneously block two or more RTKs or their ligands can reduce the escape of tumor cells and improve the therapeutic effect.

With stronger specificity, targeting and reduced off-target toxicity: the two antigen-binding arms of the bispecific antibody can bind to different antigens. The two antigen-binding arms are respectively combined with two antigens on the surface of cancer cells, which can effectively enhance the binding specificity and targeting of antibodies to cancer cells and reduce side effects such as off-target.

Effectively reducing the cost of treatment: Taking BiTE as an example, compared with traditional antibodies, has strong competitiveness in tissue penetration, tumor cell killing efficiency, off-target rate and clinical indications and other indicators, and has significant clinical advantages. Especially in terms of dosage, due to its therapeutic effect can reach 100-1000 times than that of ordinary antibodies, the lowest dosage can be 1/2000 of the original, thereby significantly reducing the cost of drug treatment. Relative to combination therapy, the cost of bispecific antibodies is also much lower than combination therapy of two single agents.

At present, there are three bispecific antibody drugs approved for marketing in the world:
In 2009, the first therapeutic bispecific antibody-Catumaxomab of TrionPharma (targeting CD3 and EpCAM) was approved by the European Union for the treatment of malignant ascites. In 2014, FDA quickly approved the bispecific antibody drug Blinatumomab (targeting CD3 and CD19) developed by Amgen based on BiTE^{®}Technology for the treatment of acute B lymphoblastic leukemia, and it is also the first approved drug targeting CD19. Amgen has owned a dozen types of BitE^{®} molecules in clinical development of a series of hematological malignancies and solid tumors. AMG420 targeting BCMA/CD3 has been granted fast track approval status by FDA. In the treatment of solid tumors, the clinical trial results of the BiTE^{®} molecule AMG212 (Pasotuxizumab) targeting prostate-specific membrane antigen (PSMA) have shown its edge. In November 2017, FDA quickly approved the bispecific antibody Emicizumab (targeting coagulation factor X and factor IXa) of Roche for the treatment of hemophilia. In December 2018, it was approved for marketing in China and was the first bispecific antibody approved in China.

Bispecific antibodies have many advantages and can be used in many therapeutic fields, such as cancers, chronic inflammatory diseases, autoimmune diseases, and infections, etc. However, the main technical difficulty in the production of bispecific antibodies is to obtain correctly paired bispecific antibodies, especially for asymmetric bispecific antibodies comprising Fc regions (IgG-like), while facing the problem of HC/HC and LC/HC mismatch at the same time. KiH, ART-Ig and BiMab technologies are subsequently developed to reduce HC/HC mismatch. While CrossMAb technology (one pair of heavy chain and light chain variable domains VL and VH are replaced with each other, while constant domains CL and CH1 also are replaced with each other), YBODY technology (on one side, a traditional Fab is used to form a monovalent unit Fab-Fc targeting antigen A, on the other side, a single chain antibody is used to form a single chain unit ScFv-Fc targeting antigen B), WuXiBody (heavy chain constant region CH1 and light chain constant region CL are replaced with TCR constant region) and common light chain technology are used to reduce the mismatch of LC/HC. However, in the field of bispecific antibodies, it is still necessary to further develop new technologies to solve the problem of LC/HC mismatch.

### 1. Her2

Members of the ErbB family of receptor tyrosine kinases are important mediators for cell growth, differentiation and survival. This receptor family comprises four unique members, including epidermal growth factor receptor (EGFR or ErbB 1), Her2 ( ErbB2), Her3 (ErbB3), and Her4 (ErbB4 or tyro2). Her2 is a transmembrane, surface-bound receptor tyrosine kinase and is normally involved in signal transduction pathways leading to cell growth and differentiation.

Overexpression of Her2 may lead to dysfunction of normal cells, and is usually closely related to the occurrence and development of tumors. Homologous or heterologous polymerization of Her2 can lead to phosphorylation of receptor tyrosine residues and initiate many signaling pathways and cause cell proliferation and tumorigenesis. As a prognostic and predictive biomarker, amplification or overexpression of the Her2 gene occurs in about 15-30% of breast cancers and 10-30% of gastric/esophageal cancers. Overexpression of Her2 can also be observed in other tumors such as ovarian, endometrial, bladder, lung, colon, and head and neck tumors.

Trastuzumab recognizes the proximal membrane epitope of Her2 extracellular domain IV. Specifically, it is an epitope consisting of three loops (557-561, 570-573, and 593-603) at the C-terminus of the portion of the Her2 extracellular domain IV. Because the epitope can be close to or interact directly with the binding domain of its dimerization partner, the binding of trastuzumab to the epitope can induce steric hindrance that inhibits the dimerization process. In addition, the binding of trastuzumab may also protect the extracellular domain of the Her2 receptor from hydrolysis attacked by proteases.

At present, trastuzumab is used as a first-line drug for the treatment of breast cancer, which is effective in the treatment of metastatic breast cancer overexpressing Her2, and the objective response rate of the first-line treatment of single drug is 30-50%; however, the effect in the treatment of metastatic breast cancer with low Her2 expression is not ideal, and the antibody initially develops resistance in many patients where the antibody is initially effective within 1 year. Her2 together with other members of the family (Her1, Her3 and Her4) can form ligand-dependent or ligand-independent heterodimers, thereby activating downstream pathways and then leading to proliferation of tumor cells, while trastuzumab cannot inhibit the formation of heterodimers, so this may be one of the reasons for the development of drug resistance.

Pertuzumab is a humanized monoclonal antibody specifically designed to prevent the HER2 receptor from pairing (dimerizating) with other HER receptors (EGFR/HER1, HER3 and HER4) on the cell surface, which is a process thought to play a role in tumor growth and survival. Pertuzumab has a certain therapeutic effect on advanced prostate cancer, non-small cell lung cancer, ovarian cancer and breast cancer, but its therapeutic effect also depends on the expression level of Her2. Pertuzumab recognizes the key site for Her2 extracellular domain II heterodimerization, and the epitope thus recognized is located in the central region 245-311 of the subregion II, and the key residues are H245, V286, S288, L295, H296 and K311. Among them, L295 and H296 are key sites that mediate the heterodimerization of Her2 and Her3, and the L295A/H296A double mutation can completely block the heterodimerization of Her2/Her3 (Franklin, M .C. et al., Insights into ErbB signaling from the structure of the ErbB2-pertuzumab complex. Cancer Cell, 2004. 5(4): pp. 317-28). Therefore, Pertuzumab can be used to effectively inhibit the formation of Her2/Her3 heterodimer, but does not show a significant inhibitory effect on the formation of EGFR/Her2 heterodimer.

### 2. IL15/IL15Ra

IL-15 is a cytokine with the length of 14-15 kDa, which is important for function of NK cell, NKT cell, and memory CD8⁺T cell. IL-15 is present in small amounts in the body, but by binding to its receptor IL-15Rα, a complex of IL-15 superagonist (IL-15 SA) with extremely high biological potency is produced and transported to target cells together. IL-15 SA strongly activates cells that respond to IL-15, especially NK cells, thus promoting anti-tumor and anti-viral functions.

IL-15, first identified as a T-lymphocyte growth factor in 1994, has approximately 19% homology to IL-2, and many biological properties of them are very similar. The three-dimensional structure of IL-15 is similar to IL-2, consisting of four "up-down-down-down" helical bundles, and other cytokines such as IL-4, IL-7, and IL-9 also contain this conformation. Unlike other cytokines, IL-15 receptor alpha is expressed on IL-15-producing cells (e.g., macrophages and dendritic cells) and forms IL-15SA with IL-15 to deliver signals to NK, NKT, and memory CD8⁺T cells expressing IL-15Rβ (also known as II-2Rβ) and a common γ chain (shared with IL-2,IL-4,IL-7,IL-9, and IL-21). It is likely that this unique mode of presentation endows IL-15 the ability to mediate its unique function. Mouse IL-15 has 70% amino acid sequence homology with human IL-15. Human IL-15 and mouse IL-15 also have similar *trans* expression patterns, signaling pathways, and biological activities. IL-15 is expressed in many cell types and tissues, including monocytes, macrophages, DCs, keratinocytes, fibroblasts, muscle cells, and neural cells. As a pleiotropic cytokine, IL-15 plays an important role in innate and adaptive immunity.

The trans-expressed IL-15/IL-15Rα signal induces the recruitment and activation of JAK1 and JAK3 by responding to the β and γ chains expressed on the cell, and the activated JAK1 and JAK3 further phosphorylate STAT3 and STATS. STAT3 and STATS are phosphorylated to form a homodimer, which translocate to the nucleus and promote the transcription of target genes. IL-15 signaling stimulates a range of downstream responses, inducing cell growth, reducing apoptosis, and enhancing the activation and metastasis of immune cells. In the absence of high affinity IL-15Rα, IL-15 can also bind to the β and γ receptor complex (IL-15Rβγ) with intermediate affinity (Ka = 1.10⁹/M) alone, induce phosphorylation and activation of other tyrosine kinases (such as Lck, Fyn, Lyn, Syk), and interact with PI3K and MAPK pathways. Studies have shown that the metabolic checkpoint kinase mTOR can also be activated by high concentrations of IL-15, which is related to the proliferation and activation of NK cells: selective knockout of mTOR will lead to delayed maturation of bone marrow NK cells. The ability of IL-15 to promote NK cell proliferation is due in part to IL-15-mediated aerobic glycolysis. The basal metabolism of NK cells is low in the absence of IL-15, but this physiological activity can be significantly enhanced by increasing IL-15 concentration.

Since IL-15 has immunological properties similar to IL-2: inducing the proliferation and survival of T cells, promoting the proliferation and differentiation of NK cells, and inducing the production of cytotoxic T lymphocytes. But unlike IL-2, IL-15 has no obvious effect on Treg cells and does not cause capillary leak syndrome in mice or non-human primates (NHP), so IL-15 is a superior choice for tumor immunotherapy compared to IL-2. Rhesus IL-15 (rIL-15) is the first IL-15 form to be used for *in vivo* experiments, and the researchers believe rIL-15 can preferentially bind to IL-15Rα on the surface of cell. The heterodimer IL-15/IL-15Rα is a natural form of the IL-15 that is cleaved from cells and can be independent of the response of cellular stimuli interactions. Novartis is currently conducting clinical trials of molecule in this form (NIZ985) in solid tumors. The RLI developed by Cytune is a fusion protein consisting of a IL-15 linked to the Sushi domain of IL-15Rα, which can act as a soluble IL-15 agonist. The IL-15/IL-15Rα-Fc complex, produced by mixing a commercially available IL-15Rα-Fc chimeric fusion protein with rIL-15, has been widely used in preclinical studies.

However, the clinical use of cytokines has the disadvantage of poor targeting of single drug administration. Only high concentration administration can achieve anti-tumor effect, while high concentration administration will produce immunosuppression and high toxicity. Furthermore, the activation of immune system by non-targeted cytokines is systemic, and the immune system is broadly activated with fatal side effects. In addition, since cytokines are small molecular weight proteins and do not have the protection mechanism of antibodies *in vivo* circulation, simple cytokines tend to have a short half-life and require repeated high-dose administration in a short period of time. At present, PEGylation or Fc fusion is mostly used in clinical research to improve the half-life of cytokines. Although the half-life is prolonged, the problem of poor targeting of cytokines still cannot be solved.

### SUMMARY OF THE PRESENT APPLICATION

The first aspect of the present invention provides a bispecific antibody, comprising: a) a light chain and a heavy chain of a first antibody that specifically binds to a first antigen; and b) a light chain and a heavy chain of a second antibody that specifically binds to a second antigen; wherein IL15 and IL15Rα are introduced into the two chains of the first antibody (e.g., CL1 and CH1 are replaced by IL15 and IL15Rα respectively), and the IL15/IL15Rα complex is formed by employing the high affinity of IL15 and IL15Rα, thereby achieving the correct pairing of the light chain/heavy chain of the first antibody and solve the problem of light chain/heavy chain mismatch of the bispecific antibody. At the same time, by mutating the amino acid sequences of the variable domains VH1, VL1, IL15 and IL15Rα of the first antibody, one or more pairs of disulfide bonds between VH1 and VL1 and between IL15 and IL15Rα are added, and the binding activity between the light chain/heavy chain of the first antibody is further enhanced, thereby effectively overcoming the problem of light chain/heavy chain mismatch of the bispecific antibody.

A bispecific antibody constructed by the present invention comprises: a) a light chain and a heavy chain of a first antibody that specifically binds to a first antigen; and b) a light chain and a heavy chain of a second antibody that specifically binds to a second antigen; wherein the two chains of the first antibody comprise IL15 and IL15Rα,respectively, and are capable of forming an IL15/IL15Rα complex.

In another preferred embodiment, the IL15 comprises a mutant capable of binding to IL15Rα,and the IL15Rα comprises a mutant capable of binding to IL15.

In another preferred embodiment, the variable domains VH1 and VL1 of the first antibody are linked or polymerized, and the IL15 and IL15Rα are linked or polymerized.

In another preferred embodiment, the constant domains CH1 and CL of the first antibody are replaced by the IL15 and IL15Rα.

In another preferred embodiment, the variable domains VL1 and VH1 of the first antibody are linked to the N-terminus of IL15 and IL15Rα.

In another preferred embodiment, the variable domains VL1 and VH1 of the first antibody are linked to the C-terminus of IL15 and IL15Rα.

In another preferred embodiment, the positions of the variable domains VL1 and VH1 of the first antibody are exchanged.

In another preferred embodiment, the light chain of the first antibody (from the N-terminus to the C-terminus) and the heavy chain of the first antibody (from the N-terminus to the C-terminus) are polymerized in the same direction.

In another preferred embodiment, the light chain of the first antibody (from the C-terminus to the N-terminus) and the heavy chain of the first antibody (from the N-terminus to the C-terminus) are polymerized in a reverse direction.

In another preferred embodiment, the variable domains VL1 and VH1 of the first antibody are linked to IL15 and IL15Rα by the amino acid linker sequence with low immunogenicity.

In another preferred embodiment, there are one or more pairs of disulfide bonds between IL15 and IL15Rα.

In another preferred embodiment, the IL15 comprises the following mutations, the counting method is that the first amino acid of IL15 as shown in SEQ ID No.1 is counted as the 1st position.

| Combination number | **IL15 mutations** |
|---|---|
| 1 | N1D |
| 2 | N4D |
| 3 | D8N |
| 4 | D30N |
| 5 | D61N |
| 6 | E64Q |
| 7 | N65D |
| 8 | Q108E |
| 9 | N1D/D61N |
| 10 | N1D/E64Q |
| 11 | N4D/D61N |
| 12 | N4D/E64Q |
| 13 | D8N/D61N |
| 14 | D8N/E64Q |
| 15 | D61N/E64Q |
| 16 | E64Q/Q108E |
| 17 | N1D/N4D/D8N |
| 18 | D61N/E64Q/N65D |
| 19 | N1D/D61N/E64Q/Q108E |
| 20 | N4D/D61N/E64Q/Q108E |

In another preferred embodiment, the IL15 and IL15Rα comprise the following mutation combinations, and the counting method is that the first amino acid of IL15 as shown in SEQ ID No.1 is counted as the 1st position; the first amino acid of IL15Rα as shown in SEQ ID No.3 is counted as the 1st position.

| **Combination number** | **IL15** | **IL15Ra** |
|---|---|---|
| 1 | wt | D96 |
| 2 | wt | D96/P97 |
| 3 | wt | D96/P97/A98 |
| 4 | E87C | D96/C97 |
| 5 | E87C | D96/P97/C98 |
| 6 | E87C | D96/C97/A98 |
| 7 | V49C | S40C |
| 8 | L52C | S40C |
| 9 | E89C | K34C |
| 10 | Q48C | G38C |
| 11 | E53C | L42C |
| 12 | C42S | A37C |
| 13 | L45C | G38C |
| 14 | L45C | A37C |

In another preferred embodiment, there are one or more pairs of disulfide bonds between the variable domains VH1 and VL1 of the first antibody.

In another preferred embodiment, the VH1 and VL1 comprise the following mutation combination forms, according to EU numbering.

| | **Modification site of disulfide bond** | |
|---|---|---|
| | VH | VL |
| Combination 1 | 37C | 95C |
| Combination 2 | 44C | 100C |
| Combination 3 | 44C | 105C |
| Combination 4 | 45C | 87C |
| Combination 5 | 100C | 50C |
| Combination 6 | 100bC | 49C |
| Combination 7 | 98C | 46C |
| Combination 8 | 101C | 46C |
| Combination 9 | 105C | 43C |
| Combination 10 | 106C | 57C |

In another preferred embodiment, the heavy chain of the first antibody and the heavy chain of the second antibody comprise a chain A and a chain B with different mutations in the Fc segment, respectively, and the chain A and the chain B have the following mutation combination forms according to EU numbering.

| Combination number | FC | Heterodimer mutation (Eu numbering) |
|---|---|---|
| 1 | FC-A chain | T366Y |
| | FC-B chain | Y407T |
| 2 | FC-A chain | T366W |
| | FC-B chain | T366S/L368A/Y407V |
| 3 | FC-A chain | S354C/T366W |
| | FC-B chain | Y349C/T366S/L368A/Y407V |
| 4 | FC-A chain | S364H/F405A |
| | FC-B chain | Y349T/T394F |
| 5 | FC-A chain | T350V/L351 Y/F405A/Y407V |
| | FC-B chain | T350V/T366L/K392L/T394W |
| 6 | FC-A chain | K392D/K409D |
| | FC-B chain | E356K/D399K |
| 7 | FC-A chain | D221 E/P228E/L368E |
| | FC-B chain | D221 R/P228R/K409R |
| 8 | FC-A chain | K360E/K409W |
| | FC-B chain | Q347R/D399V/F405T |
| 9 | FC-A chain | K360E/K409W/Y349C |
| | FC-B chain | Q347R/D399V/F405T/S354C |
| 10 | FC-A chain | K370E/K409W |
| | FC-B chain | E357N/D399V/F405T |
| 11 | FC-A chain | F405L |
| | FC-B chain | K409R |
| 12 | FC-A chain | K360D/D399M/Y407A |
| | FC-B chain | E345R/Q347R/T366V/K409V |
| 13 | FC-A chain | Y349S/K370Y/T366M/K409V |
| | FC-B chain | E356G/E357D/S364Q/Y407A |
| 14 | FC-A chain | L351 D/L368E |
| | FC-B chain | L351 K/T366K |
| 15 | FC-A chain | GQPFRPEVHLLPPSREEMTKNQVSLTCLARGFYPKDIAVEWES NGQPENNYKTTPSRQEPSQGTTTFAVTSKLTVDKSRWQQGNV FSCSVMHEALHNHYTQKTISL |
| | FC-B chain | GQPREPQVYTLPPPSEELALNELVTLTCLVKGFYPSDIAVEWLQ GSQELPREKYLTWAPVLDSDGSFFLYSILRVAAEDWKKGDTFS CSVMHEALHNHYTQKSLDR |
| 16 | FC-A chain | L368D/K370S |
| | FC-B chain | E357Q/S364K |
| 17 | FC-A chain | S354C/T366W/K409A |
| | FC-B chain | Y349C/T366S/L368A/Y407V/F405K |
| 18 | FC-A chain | S354C/T366W/F405K/K360EQ347E |
| | FC-B chain | Y349C/T366S/L368A/Y407V/Q347R/T394W |
| 19 | FC-A chain | T366W/K409A |
| | FC-B chain | T366S/L368G/Y407A/F405K |
| 20 | FC-A chain | knobs(T366W/F405K) |
| | FC-B chain | holes(T366S/L368G/Y407A/K409A) |
| 21 | FC-A chain | Q347A/S364K/T366V/K370T/K392Y/F405S/Y407V/K409W/T411 N |
| | FC-B chain | Q347E/Y349A/L351F/S364T/T366V//K370T/T394D/V397L/D399E /D401Q/F405A/Y407S/K409R/T411R |
| 22 | FC-A chain | K274Q/N276K/Y300F/A339T/Q347A/S364K/T366V/K370T/N384S /K392Y/V397M/F405S/Y407V/K409W/T411N/V422I/H435R/Y436 F |
| | FC-B chain | Q347E/Y349A/L351F/S364T/T366V//K370T/T394D/V397L/D399E /D401Q/F405A/Y407S/K409R/T411R |

In another preferred embodiment, the Fc segment comprises Human IgG1 Fc, Human IgG2 Fc, Human IgG3 Fc, Human IgG4 Fc and variants thereof.

In another preferred embodiment, among the chain A and chain B of the Fc segment, one chain can bind to protein A, the other chain is a variant that is not able to bind to protein A, and the mutation comprises H435R or H435R/Y436F, according to EU numbering.

In another preferred embodiment, the first antigen is any one of CD3, CD20, CD19, CD30, CD33, CD38, CD40, CD52, slamf7, GD2, CD24, CD47, CD133, CD217, CD239, CD274, CD276, PD-1, CEA, Epcam, Trop2, TAG72, MUC1, MUC16, mesothelin, folr1, CLDN18.2, PDGFR2,FVIII, C- MET, EGFR, EGFR, SCA ephA2, ADAM17, 17-A1, NKG2D ligands, MCSP, LGR5, SSEA3, SLC34A2, BCMA, GPNMB, IL-6R, IL-2R, CCR4, VEGFR-2, CD6, CTLA-4, integrin α4, DNA/histone complex, PDGFRα, NeuGcGM3, IL-4Rα, IL-6Rα, the second antigen is a different epitope of the first antigen or another antigen as described above.

In another preferred embodiment, the first/second antibody is a chimeric, humanized or fully human antibody.

In another preferred embodiment, the bispecific antibody has a structure shown in Formula I: 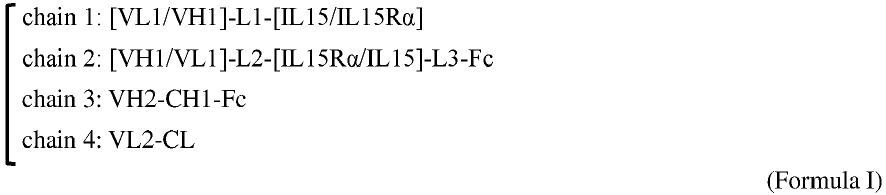 wherein,
chain 1: VL1 or VH1 is linked to the N- or C-terminus of IL15 or IL15Rα via L1;
chain 2: arranged from N-terminus to C-terminus, VH1 or VL1, L2, IL15Rα or IL15, L3, Fc;
chain 3: the heavy chain of the second antibody is arranged from the N-terminus to the C-terminus, VH2-CH1-Fc;
chain 4: the light chain of the second antibody is arranged from the N-terminus to the C-terminus, VL2-CL;
"-" represents a peptide bond;
L1, L2 and L3 are each independently a bond or a linker sequence;

In another preferred embodiment, the bispecific antibody has a structure shown in Formula II: 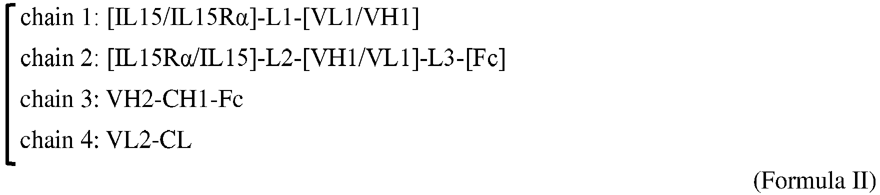 wherein,
chain 1: IL15 or IL15Rα is linked to the N- or C-terminus of VL1 or VH1 via L1;
chain 2: arranged from N-terminus to C-terminus, IL15Rα or IL15, L2, VH1 or VL1, L3, Fc;
chain 3: the heavy chain of the second antibody is arranged from the N-terminus to the C-terminus, VH2-CH1-Fc;
chain 4: the light chain of the second antibody is arranged from the N-terminus to the C-terminus, VL2-CL;
"-" represents a peptide bond;
L1, L2 and L3 are each independently a bond or a linker sequence.

In another preferred embodiment, the chain 1 and chain 2 comprise the following combination forms:

| | | |
|---|---|---|
| Combination 1 | Chain 1: | VL1-L1-IL15 |
| | Chain 2: | VH1-L2-IL15Ra-L3-Fc (chain A or B) |
| Combination 2 | Chain 1: | IL15-L1-VL1 |
| | Chain 2: | VH1-L2-IL15Ra-L3-Fc (chain A or B) |
| Combination 3 | Chain 1: | VL1-L1-IL15 |
| | Chain 2: | IL15Ra-L2-VHl-L3-Fc (chain A or B) |
| Combination 4 | Chain 1: | IL15-L1-VL1 |
| | Chain 2: | IL15Ra-L2-VHl-L3-Fc (chain A or B) |
| Combination 5 | Chain 1: | VH1-L1-IL15 |
| | Chain 2: | VL1-L2-IL15Ra-L3-Fc (chain A or B) |
| Combination 6 | Chain 1: | IL15-L1-VH1 |
| | Chain 2: | VL1-L2-IL15Ra-L3-Fc (chain A or B) |
| Combination 7 | Chain 1: | VH1-L1-IL15 |
| | Chain 2: | IL15Ra-L2-VLl-L3-Fc (chain A or B) |
| Combination 8 | Chain 1: | IL15-L1-VH1 |
| | Chain 2: | IL15Ra-L2-VLl-L3-Fc (chain A or B) |
| Combination 9 | Chain 1: | VL1-L1-IL15Ra |
| | Chain 2: | VH1-L2-IL15-L3-Fc (chain A or B) |
| Combination 10 | Chain 1: | IL15Ra-Ll-VLl |
| | Chain 2: | VH1-L2-IL15-L3-Fc (chain A or B) |
| Combination 11 | Chain 1: | VL1-L1-IL15Ra |
| | Chain 2: | IL15-L2-VH1-L3-Fc (chain A or B) |
| Combination 12 | Chain 1: | IL15Ra-Ll-VLl |
| | Chain 2: | IL15-L2-VH1-L3-Fc (chain A or B) |
| Combination 13 | Chain 1: | VH1-L1-IL15Ra |
| | Chain 2: | VL1-L2-IL15-L3-Fc (chain A or B) |
| Combination 14 | Chain 1: | IL15Ra-Ll-VHl |
| | Chain 2: | VL1-L2-IL15-L3-Fc (chain A or B) |
| Combination 15 | Chain 1: | VH1-L1-IL15Ra |
| | Chain 2: | IL15-L2-VL1-L3-Fc (chain A or B) |
| Combination 16 | Chain 1: | IL15Ra-Ll-VHl |
| | Chain 2: | IL15-L2-VL1-L3-Fc (chain A or B) |

In another preferred embodiment, the bispecific antibody has a structure shown in Formula III: 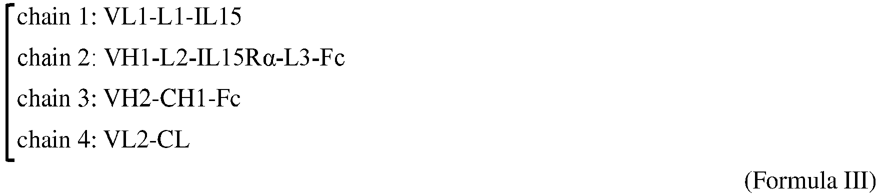 wherein,
chain 1: arranged from N-terminus to C-terminus, VL1-L1-IL15, L1 is a low immunogenicity amino acid linker sequence;
chain 2: arranged from N-terminus to C-terminus, VH1-L2-IL15Rα-L3-Fc, L2 or L3 is a low immunogenicity amino acid linker sequence;
chain 3: arranged from N-terminus to C-terminus, VH2-CH1-Fc;
chain 4: arranged from N-terminus to C-terminus, VL2-CL;
"-" represents a peptide bond.

In another preferred embodiment, the L1, L2 and L3 comprise glycine (G) and serine (S) residues.

In another preferred embodiment, the L1, L2 and L3 comprise one or more GGGGS repeats.

In another preferred embodiment, the IL15 sequence is shown in SEQ ID No. 1 or SEQ ID No.2; the IL15Rα sequence is shown in SEQ ID No.3, SEQ ID No.4, SEQ ID No.5, SEQ ID No.6, SEQ ID No.7, SEQ ID No.8 or SEQ ID No.9.

In another preferred embodiment, the Fc sequence is shown in SEQ ID No. 18 or SEQ ID No. 19.

In another preferred embodiment, the first antigen and the second antigen are two antigens binding to two different epitopes of Her2 respectively.

In another preferred embodiment, the bispecific antibody is obtained by expressing fusion sequences of SEQ ID No.10, SEQ ID No. 11, SEQ ID No. 13, SEQ ID No.12 .

In another preferred embodiment, the first antigen and the second antigen are CS 1 antigen and CD38 antigen, respectively.

In another preferred embodiment, the bispecific antibody is obtained by expressing fusion sequences of SEQ ID No.14, SEQ ID No.15, SEQ ID No.17, SEQ ID No.16.

The second aspect of the present invention provides a pharmaceutical composition comprising:
(a) the bispecific antibody of the first aspect of the present invention; and
(b) a pharmaceutically acceptable carrier.

In another preferred embodiment, the pharmaceutical composition further comprises other drugs for treating cancer (or tumor), such as chemotherapeutic drugs.

The third aspect of the present invention provides a use of the bispecific antibody of the first aspect of the present invention and the pharmaceutical composition of the sixth aspect of the present invention in the preparation of a medicament for treating cancer (or tumor), infectious or immunomodulatory disease.

The fourth aspect of the present invention provides a use of the bispecific antibody of the first aspect of the present invention in the preparation of a medicament for inhibiting tumor growth.

In another preferred embodiment, the cancer or tumor comprises cancer or tumor from the following sites: colorectal, breast, ovary, pancreas, stomach, prostate, kidney, cervix, thyroid, endometrium, uterus, bladder, neuroendocrine, head and neck, liver, nasopharynx, testis.

In another preferred embodiment, the cancer (or tumor) includes: myeloma, lymphoma, leukemia, small cell lung cancer, non-small cell lung cancer, melanoma, basal cell skin cancer, squamous cell skin cancer, carina Dermatofibrosarcoma, Merkel cell carcinoma, glioblastoma, glioma, sarcoma, mesothelioma, and myelodysplastic syndrome.

The fifth aspect of the present invention provides a use of the bispecific antibody of the first aspect of the present invention binding to double epitopes of Her in the preparation of a reagent or kit for diagnosing a HER2 positive tumor (such as breast cancer and gastric cancer).

### Beneficial effects of the invention

Through experiments, the inventors of the present invention surprisingly found that a bispecific antibody introduces IL15 and IL15Rα into the two chains of the first antibody (for example, CL1 and CH1 were replaced with L15 and IL15Rα, respectively), and the IL15/IL15Rα complex was formed by employing the high affinity of IL15 and IL15Rα, thereby realizing the correct pairing of the light chain/heavy chain of the first antibody and solving the problem of mismatch of light chain/heavy chain of bispecific antibody. At the same time, by mutating the amino acid sequences of the variable domains VH1, VL1, IL15 and IL15Rα of the first antibody, one or more pairs of disulfide bonds are added between VH1 and VL1 and between IL15 and IL15Rα, and the binding activity between the light chain/heavy chain of the first antibody is further enhanced, thereby effectively overcoming the challenges of mismatch of light chain/heavy chain, high by-products, and poor stability in the preparation of bispecific antibodies, and ultimately a cytokine-containing, bispecific targeting, and correctly paired multifunctional antibody is prepared. At the same time, the development cycle of bispecific antibodies is shortened and the production cost is reduced.

On the other hand, the bispecific antibody constructed by the present invention has IL-15/IL-15Rα activity while overcoming the problem of light chain/heavy chain mismatch. It can target cytokines to the tumor site, specifically expand and activate T cells and NK cells in PMBC at the tumor site, and can increase the number of immune cells and the release of killer cytokines, which can more effectively kill tumor cells, and reduce the administration dosage.

In the third aspect, the present invention successfully constructs a correctly paired Trastuzumab/Pertuzumab/IL15 bispecific antibody.IL15 is targeted to tumor tissue by employing the targeting of Her2 bispecific antibody, immune response is stimulated, which can kill Her2 positive tumor by multiple mechanisms.

In the fourth aspect, the present invention successfully constructs a correctly paired Elotuzumab/Daratumumab/IL15 bispecific antibody that binds to CS1/CD38 antigens. The bispecific antibody has a CS1 antigen binding ability equivalent to CS1 monoclonal antibody, and a CD38 antigen binding ability equivalent to CD38 monoclonal antibody, which has great potential in the treatment of blood tumors.

In order to more easily understand the present invention, certain technical and scientific terms are specifically defined below. Unless clearly defined otherwise elsewhere in this document, all other technical and scientific terms used herein have the meaning commonly understood by those of ordinary skill in the art to which the present invention belongs.

### Term

The three-letter and one-letter codes for amino acids used in the present invention are as described in J.Boil.Chem., 243, p3558 (1968).

The "antibody" of the present invention includes not only intact antibodies, but also fragments, polypeptide sequences, derivatives and analogs thereof with antigen-binding activity.

The antigen-binding fragment refers to one or more portions of a full-length antibody that retain the ability to bind to an antigen (e.g., HER2) and compete with the intact antibody for specific binding to the antigen. See generally, Fundamental Immunology, Ch. 7 (Paul, W., ed., 2nd edition, Raven Press, N.Y. (1989), which is incorporated by reference in its entirety for all purposes. Antigen-binding portions can be produced by recombinant DNA techniques or by enzymatic or chemical cleavage of intact antibodies. In some cases, the antigen-binding portion includes a Fab, Fab', F(ab')2, Fd, Fv, dAb, and a complementarity determining region (CDR) fragment, a single chain antibody (e.g., scFv), a chimeric antibody comprising at least a portion of an antibody sufficient to confer specific antigen binding ability to the polypeptide. Conventional techniques known to those skilled in the art (such as recombinant DNA technology or enzymatic or chemical cleavage methods) can be used to obtain an antigen binding portion (such as the antibody fragment described above) of an antibody from a given antibody (such as monoclonal antibody 2E12), and to specifically screen the antigen binding portion of the antibody in the same manner as for an intact antibody. The term "Fd fragment" means an antibody fragment consisting of the VH and CH1 domains; the term "Fv fragment" means an antibody fragment consisting of the VL and VH domains of a single arm of an antibody; the term "dAb fragment" means an antibody fragment consisting of the VH domain (Ward et al., Nature 341: 544-546 (1989)); the term "Fab fragment" means an antibody fragment consisting of VL, VH, CL and CH1 domains; the term "F(ab')2 fragment" means an antibody fragment comprising two Fab fragments connected by a disulfide bridge on the hinge region.

As used herein, the terms "fragment", "derivative" and "analog" refer to a polypeptide that substantially retain the same biological function or activity of the antibody of the present invention. The polypeptide fragment, derivative or analog of the present invention may be (i) a polypeptide with one or more conservative or non-conservative amino acid residues (preferably conservative amino acid residues) substituted, and such substituted amino acid residues may or may not be encoded by the genetic code, or (ii) a polypeptide with a substituent group in one or more amino acid residues, or (iii) a polypeptide formed by fusion of a mature polypeptide with another compound (such as a compound that extends the half-life of the polypeptide, such as polyethylene glycol), or (iv) a polypeptide formed by fusion of an additional amino acid sequence to the polypeptide sequence (such as a leader sequence or secretory sequence or sequence or protein sequence used to purify the polypeptide, or a fusion protein formed with a 6His tag). According to the teachings herein, these fragments, derivatives and analogs are within the scope well-known to those skilled in the art.

The term "epitope" or "antigenic epitope" as used herein refers to a site on an antigen that is specifically bound by an immunoglobulin or antibody. An "epitope" is also referred to an "antigenic determinant" in the art. An epitope or antigenic determinant typically consists of chemically active surface groups of molecules such as amino acids or carbohydrates or sugar side chains and typically has a specific three-dimensional structural characteristic as well as specific charge characteristics. For example, an epitope typically includes at least 3, 4, 5, 6, 7, 8, 9, 10, 11, 12, 13, 14, or 15 contiguous or non-contiguous amino acids in a unique spatial conformation, which may be "linear" or "conformational". See, e.g., Epitope Mapping Protocols in Methods in Molecular Biology, vol. 66, G.E. Morris, Ed. (1996). In a linear epitope, all points of interaction between a protein and an interacting molecule (e.g., an antibody) exist linearly along the primary amino acid sequence of the protein. In a conformational epitope, points of interaction exist across protein amino acid residues that are separated from each other.

The "IL-15" of the present invention may be any IL-15 or a mutant capable of binding to IL15Rα,such as a human IL-15 or a non-human mammalian or non-mammalian IL-15. Exemplary non-human mammals comprise such as pigs, rabbits, monkeys, orangutans, mice, and the like, non-mammals such as chickens, and the like; preferably human IL-15. The term "a mutant capable of binding to IL15Rα" refers to a mutant molecule obtained by mutation of one or more amino acid substitutions, additions or deletions, with an increased or decreased affinity for IL-15 and its receptor, or with an increased or decreased activity of stimulating T cells or NK cells. Preferably, the "IL-15" of the present invention is its variant form, more preferably the amino acid sequence of that is SEQ ID No.1 or SEQ ID No.2.

The "IL-15Rα" of the present invention may be IL-15Rα of any species or a mutant capable of binding to IL15Rα,such as human IL-15Rα or non-human mammalian IL-15Rα or non-mammalian IL-15Rα. Exemplary non-human mammals comprise such as pigs, rabbits, monkeys, orangutans, mice, and the like, non-mammals such as chickens, and the like. Preferably human IL-15Rα, more preferably a human IL-15Rα extracellular domain fragment, which is abbreviated as IL-15Rα ECD (see database UniProtKB, accession number Q13261, 31-205aa). The term "a mutant capable of binding to IL15Rα" refers to a functional mutant formed by one or more amino acid deletion, insertion or substitution on IL-15Rα and has the ability to bind to its ligand molecule such as IL-15, preferably a human IL-15Rα molecule, more preferably a truncated form of a human IL-15Rα extracellular domain fragment, that is, a molecule with human IL-15 receptor α activity obtained by deletion of one or more amino acids starting from the C-terminal of the extracellular domain fragment, preferably retaining the deletion mutation form of 65-178 amino acids, such as IL-15Rα (SEQ ID NO: 3-9).

The "Fc segment" of the present invention refers to the C-terminal region of an immunoglobulin, which has no antigen-binding activity. It is a site where an antibody molecule interacts with an effector molecule and a cell, and is a dimer molecule comprising two disulfide-linked antibody heavy chain Fc region polypeptides. The Fc region can be generated by papain digestion or IdeS digestion into a trypsin of an intact (full-length) antibody or can be produced by recombination. The "Fc portion" preferably includes at least one immunoglobulin hinge region, as well as the CH2 and CH3 regions of IgG.

Fc heterodimer mutations refer to changes in the structure or function of Fc caused by the presence of one or more amino acid substitutions, insertions, or deletions at suitable sites in Fc. The space filling effect, electrostatic steering, hydrogen bonding and hydrophobic interaction can be formed between the Fc variants designed by mutation. The interaction between Fc mutants contributes to the formation of stable heterodimers. A preferred mutation design is a "Knob-in-hole" format. In addition, the Fc of the present invention may also have other mutations that cause changes in its function, such as glycosylation modification mutations, mutations in the FcyR binding region (to adjust ADCC activity), and amino acid mutations that improve antibody stability, etc. In the present invention, Fc comprises Human IgG1 Fc, Human IgG2 Fc, Human IgG3 Fc, Human IgG4 Fc and mutants thereof, wherein one chain is capable of binding to protein A and the other chain is a mutant incapable of binding to protein A, comprising mutation H435R or H435R/Y436F, according to EU numbering.

The "heterodimer" of the present invention is preferably the product of gene co-expression. For example, it is co-expressed in prokaryotic cells, such as *E. coli;* or co-expressed in eukaryotic cells, such as 293, CHO. The term "co-express" refers to the expression of multiple genes together in a cell, with their products appearing simultaneously. These genes may be simultaneously present and controlled for expression separately or jointly. In the present invention, co-expression in one eukaryotic cell is preferred. The gene expression product obtained by co-expression facilitates efficient and simple formation of a complex; in the present invention, it facilitates the formation of a heterodimer.

The mutation design technology of Fc variant has been widely applied in the art to prepare bispecific antibody or heterodimeric Fc fusion protein form. Representative forms comprise the "Knob-in-Hole" form proposed by Cater et al. (Protein Engineering Vol. 9No.7pp617-621, 1996); the heterodimer form containing Fc formed by technicians of Amgen company using electrostatic steering (US2010286374A1); the heterodimer form formed by IgG/IgA chain exchange (SEED bodies) proposed by Jonathan H. Davis et al. (Protein Engineering, Design & Selectionpp. 1-8, 2010); the bispecific molecule formed by Genmab DuoBody (Science, 2007. 317 (5844)) platform technology; the heterodimer protein form formed by technicians of Xencor Company by combining structural calculation and Fc amino acid mutation and different action models (mAbs3:6, 546-557; November/December2011); the heterodimer protein form obtained by Fc modification method based on charge network of Suzhou Corning Jerry Company (CN 201110459100.7); and other genetic engineering methods for the formation of heterodimeric functional proteins based on changes in Fc amino acids or functional modification methods. The Knob-in-Hole structure on the Fc variant fragment of the present invention refers to the mutation of each of the two Fc fragments, which can be combined in the form of "Knob-in-Hole" after mutation. Preferably, the "Knob-in-Hole" model of Cater et al. is used to perform site mutation in the Fc region so that the obtained first Fc variant and second Fc variant can join in the form of "Knob-in-Hole" to form a heterodimer. To select a particular immunoglobulin Fc region from a particular immunoglobulin class and subclass is within the purview of one skilled in the art. The Fc regions of human antibodies IgG1, IgG2, IgG3, and IgG4 are preferred, and the Fc regions of human antibodies IgG1 and IgG4 are more preferred. One of the first Fc variant or the second Fc variant is randomly selected to make a knob mutation and the other to make a hole mutation. In examples, the first Fc variant is of knob mutation; the second Fc variant is of hole mutation.

The term "linker sequence" refers to one or more amino acid residues inserted into the immunoglobulin domain to provide sufficient mobility for the domains of the light and heavy chains to fold into the exchange dual variable region immunoglobulin. It is useful in the present invention to link IL-15 or IL-15Rα to the corresponding light or heavy chain to ensure proper protein folding and peptide stability. The "linker peptide" of the present invention is preferably consisting of low immunogenic amino acid residues, preferably (GGGGS)n, wherein n may be 0, 1, 2, 3, 4, 5, or more, preferably n is 1-5.

The antibody of the present invention can be used alone, or can be combined or coupled with a detectable label (for diagnostic purposes), a therapeutic agent, a PK (protein kinase) modifying moiety, or any combination of these substances.

A detectable label for diagnostic purposes includes, but is not limited to, a fluorescent or luminescent label, a radioactive label, a MRI (magnetic resonance imaging) or CT (Computed X-ray Tomography Technique) contrast agent, or an enzyme capable of producing a detectable product.

### DESCRIPTION OF THE DRAWINGS

Fig. 1 is the structural form 1 of a bispecific antibody.
Fig. 2 is the structural form 2 of a bispecific antibody.
Fig. 3 is the structural form 3 of a bispecific antibody.
Fig. 4 is the structural form 4 of a bispecific antibody.
Fig. 5 is the structural form 5 of a bispecific antibody.
Fig. 6 is the structural form 6 of a bispecific antibody.
Fig. 7 is the structural form 7 of a bispecific antibody.
Fig. 8 is the structural form 8 of a bispecific antibody.
Fig. 9 is the SDS-PAGE electrophoresis analysis of QP34563457 protein reducing and non-reducing bands.
Fig. 10 is the HPLC-SEC analysis of QP34563457 protein purity.
Fig. 11 is the peptide map analysis of QP34563457 protein, wherein, Fig. 11a shows 36% sequence coverage of the QP34563457 protein for the Trastuzumab VL-IL15 chain, Fig. 11b shows 56% sequence coverage of the QP34563457 protein for the Trastuzumab VH-IL15Ra-Fc chain, Fig. 11c shows 64% sequence coverage of the QP34563457 protein for the pertuzumab VL-CL chain, and Fig. 11d shows 56% sequence coverage of the QP34563457 protein for the pertuzumab VH-CH1-Fc chain.
Fig. 12 is the ELISA detection of QP34563457 and other molecules binding to Her2-Fc fusion protein.
Fig. 13 is the ELISA detection of QP34563457 and other molecules binding to Her2M2-Fc fusion protein.
Fig. 14 is the ELISA detection of QP34623463 and other molecules binding to CD38 protein.
Fig. 15 is the ELISA detection of QP34623463 and other molecules binding to CS1 fusion protein.
Fig. 16 is the FACS detection of QP34563457 and other molecules binding to SK-BR-3 cells.
Fig. 17 is the FACS detection of QP34563457 and other molecules binding to BT-474 cells.
Fig. 18 is the FACS detection of QP34563457 and other molecules binding to SK-OV-3 cells.
Fig. 19 is the proliferation inhibition curve of QP34563457 and other molecules on human breast cancer cells SK-BR-3.
Fig. 20 is the proliferation inhibition curve of QP34563457 and other molecules on human breast cancer cells BT-474.
Fig. 21 is the proliferation inhibition curve of QP34563457 and other molecules on human ovarian cancer cells SK-OV-3.
Fig. 22 shows the killing of human breast cancer cells SK-BR-3 by PBMC mediated by QP34563457 and other molecules.
Fig. 23 shows the killing of human breast cancer cells BT-474 by PBMC mediated by QP34563457 and other molecules.
Fig. 24 shows the killing of human ovarian cancer cells SK-OV-3 by PBMC mediated by QP34563457 and other molecules.
Fig. 25 shows the assay for detecting QP34563457 and other molecules on Mo7e cell proliferation.
Fig. 26 is the tumor growth curve of SK-OV-3 *in vivo* pharmacodynamic model.

### Detailed Description

The experimental methods that do not indicate specific conditions in the embodiments below are usually in accordance with conventional conditions or conditions and methods recommended by the manufacturer of raw materials or commodities. Such as Molecular Cloning, Laboratory Manual, Cold Spring Harbor laboratory, Current Molecular Biology Methods, Cell Biology and so on. Reagents without specific source are commercially available conventional reagents.

### Example 1: Cloning and expression of fusion proteins

The protein sequences are as follows:
anti Her2 (Her2 extracellular domain IV region) VL-IL15 SEQ ID NO:10 
DIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKGGGGSG
GGGSGGGGSNWVNVISDLKKIEDLIQSMHIDATLYTESDVHPSCKVTAMKCFLLE
LQVISCESGDASIHDTVENLIILANNSLSSNGNVTESGCKECEELEEKNIKEFLQSF
VHIVQMFINTS
anti Her2(Her2 extracellular domain IV region) VH-IL15Ra-Fc (Knob) SEQ ID NO:11 
EVQLVESGGGLVQPGGSLRLSCAASGFNIKDTYIHWVRQAPGKGLEWVARIYPTN
GYTRYADSVKGRFTISADTSKNTAYLQMNSLRAEDTAVYYCSRWGGDGFYAMDY
WGQGTLVTVSSGGGGSGGGGSGGGGSITCPPPMSVEHADIWVKSYSLYSRERYIC
NSGFKRKAGTCSLTECVLNKATNVAHWTTPSLKCIRGGGGSEPKSSDKTHTCPPC
PAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISK
AKGQPREPQVYTLPPSRDELTKNQVSLWCLVKGFYPSDIAVEWESNGQPENNYKT
TPPVLDSDGSFFLYSKLTVDKSRWQQGNVFSCSVMHEALHNHYTQKSLSLSPGK
anti Her2' (Her2 extracellular domain II region) VL-CL SEQ ID NO:12 
DIQMTQSPSSLSASVGDRVTITCKASQDVSIGVAWYQQKPGKAPKLLIYSASYRYT
GVPSRFSGSGSGTDFTLTISSLQPEDFATYYCQQYYIYPYTFGQGTKVEIKRTVAAP
SVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC
anti Her2' (Her2 extracellular domain II region) VH-CH1-CH2-CH3 (Hole) SEQ ID NO:13 
EVQLVESGGGLVQPGGSLRLSCAASGFTFTDYTMDWVRQAPGKGLEWVADVNP
NSGGSIYNQRFKGRFTLSVDRSKNTLYLQMNSLRAEDTAVYYCARNLGPSFYFDY
WGQGTLVTVSSASTKGPSVFPLAPSSKSTSGGTAALGCLVKDYFPEPVTVSWNSG
ALTSGVHTFPAVLQSSGLYSLSSVVTVPSSSLGTQTYICNVNHKPSNTKVDKKVEP
KSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSN
KALPAPIEKTISKAKGQPREPQVYTLPPSRDELTKNQVSLSCAVKGFYPSDIAVEWE
SNGQPENNYKTTPPVLDSDGSFFLVSKLTVDKSRWQQGNVFSCSVMHEALHNRY
TQKSLSLSPGK
anti CS1 VL-IL15 SEQ ID NO:14 
DIQMTQSPSSLSASVGDRVTITCKASQDVGIAVAWYQQKPGKVPKLLIYWASTRH
TGVPDRFSGSGSGTDFTLTISSLQPEDVATYYCQQYSSYPYTFGQGTKVEIKGGGG
SGGGGSGGGGSNWVNVISDLKKIEDLIQSMHIDATLYTESDVHPSCKVTAMKCFL
LELQVISCESGDASIHDTVENLIILANNSLSSNGNVTESGCKECEELEEKNIKEFLQS
FVHIVQMFINTS
anti CS1 VH-IL15Ra-Fc (Knob) SEQ ID NO:15 
EVQLVESGGGLVQPGGSLRLSCAASGFDFSRYWMSWVRQAPGKGLEWIGEI
NPDSSTINYAPSLKDKFIISRDNAKNSLYLQMNSLRAEDTAVYYCARPDGNYWYF
DVWGQGTLVTVSSGGGGSGGGGSGGGGSITCPPPMSVEHADIWVKSYSLYSRER
YICNSGFKRKAGTCSLTECVLNKATNVAHWTTPSLKCIRGGGGSEPKSSDKTHTCP
PCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTIS
KAKGQPREPQVYTLPPSRDELTKNQVSLWCLVKGFYPSDIAVEWESNGQPENNYK
TTPPVLDSDGSFFLYSKLTVDKSRWQQGNVFSCSVMHEALHNHYTQKSLSLSPGK
anti CD38 VL-CL SEQ ID NO:16 
EIVLTQSPATLSLSPGERATLSCRASQSVSSYLAWYQQKPGQAPRLLIYDASNR
ATGIPARFSGSGSGTDFTLTISSLEPEDFAVYYCQQRSNWPPTFGQGTKVEIKRTVA
APSVFIFPPSDEQLKSGTASVVCLLNNFYPREAKVQWKVDNALQSGNSQESVTEQ
DSKDSTYSLSSTLTLSKADYEKHKVYACEVTHQGLSSPVTKSFNRGEC
anti CD38 VH-CH1-CH2-CH3 (Hole) SEQ ID NO:17

EVQLLESGGGLVQPGGSLRLSCAVSGFTFNSFAMSWVRQAPGKGLEWVSAIS
GSGGGTYYADSVKGRFTISRDNSKNTLYLQMNSLRAEDTAVYFCAKDKILWFGEP
VFDYWGQGTLVTVSSASTKGPSVFPLAPSSKSTSGGTAALGCLVKDYFPEPVTVS
WNSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSHE
DPEVKFNWYVDGVEVHNAKTKPREEQYNSTYRVVSVLTVLHQDWLNGKEYKC
KVSNKALPAPIEKTISKAKGQPREPQVYTLPPSRDELTKNQVSLSCAVKGFYPSDIA

VEWESNGQPENNYKTTPPVLDSDGSFFLVSKLTVDKSRWQQGNVFSCSVMHEAL
HNRYTQKSLSLSPGK

### Clone construction method:

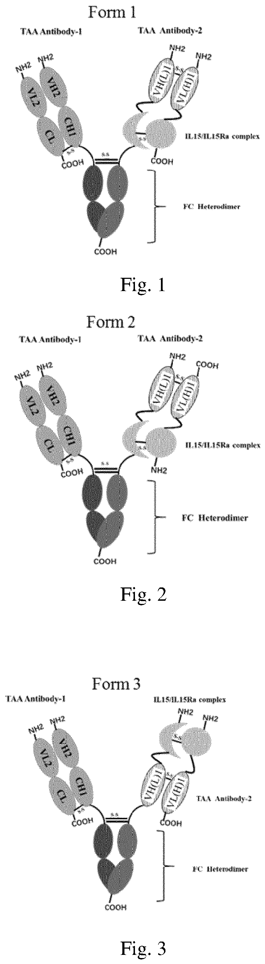

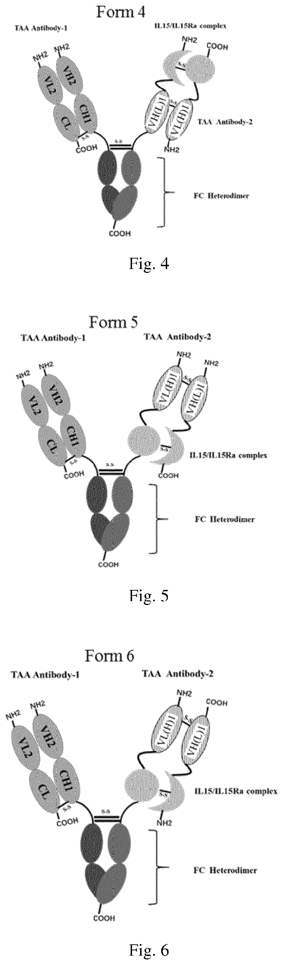

Clone designs are shown in Table 1: vectors encoding anti Her2 (Tmab) VL-IL15 (SEQ ID NO:10), anti Her2 (Tmab) VH-IL15Ra-Fc (Knob) (SEQ ID NO:11), anti CS1 VL-IL15 (SEQ ID NO:14), anti CS1 VH-IL15Ra-Fc (Knob) (SEQ ID NO:15) were constructed, respectively. The plasmid containing DHFR as a screening marker can be used for stable strain screening. Vectors were constructed to encode the light chain sequence (sequence) of the anti-tumor specific antigen antibody and the heavy chain sequence of the anti-tumor specific antigen antibody (wherein, Fc contains Hole mutation (T366S, L368A, Y407V) anti Her2' VL (Pmab)-CL (SEQ ID NO:12), anti Her2' (Pmab) VH-CH1-CH2-CH3 (Hole) (SEQ ID NO:13), anti CD38 VL-CL (SEQ ID NO:16), anti CD38 VH-CH1-CH2-CH3 (Hole) (SEQ ID NO:17), the plasmid contains GS as a screening marker which can be used for stable strain screening. A schematic structural diagram of the protein molecule is shown in FIG. 6. In other embodiments, the protein molecule may be in other structural forms, as shown in Figs. 2 to 8, respectively.

**Table 6. Construction of clone designs**

| Example Sequence | SEQ ID | Antibody Name | Clone | | |
|---|---|---|---|---|---|
| Protein-1 | SEQ ID NO:10 | QP34563457 | QD3456 | QD3452 | anti Her2 VL-IL15 |
| | SEQ ID NO:11 | | | QD3453 | anti Her2 VH-IL15Ra-Fc (Knob) |
| | SEQ ID NO:12 | | QD3457 | QD3454 | anti Her2' VL-CL |
| | SEQ ID NO:13 | | | QD3455 | anti Her2' VH-CH1-CH2-CH3 (Hole) |
| Protein-2 | SEQ ID NO:14 | QP34623463 | QD3462 | QD3458 | anti CS1 VL-IL15 |
| | SEQ ID NO:15 | | | QD3459 | anti CS1 VH-IL15Ra-Fc (Knob) |
| | SEQ ID NO:16 | | QD3463 | QD3460 | anti CD38 VL-CL |
| | SEQ ID NO: 17 | | | QD3461 | anti CD38 VH-CH1-CH2-CH3 (Hole) |

As shown in Table 7, control antibodies Trastuzumab, Pertuzumab,Elotuzumab, and Daratumumab were designed and constructed.

**Table 7. Sequences and numbering of control proteins**

| **Protein Name** | **Protein Number** | **Clone Number** | **Protein sequence** |
|---|---|---|---|
| Trastuzumab | QP32933294 | QD3293 | MEFGLSWLFLVAILKGVQCEVQLVESGGGLVQPGGSLRLSCAASGFNI KDTYIHWVRQAPGKGLEWVARIYPTNGYTRYADSVKGRFTISADTSKN TAYLQMNSLRAEDTAVYYCSRWGGDGFYAMDYWGQGTLVTVSSAST KGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPK SCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDW LNGKEYKCKVSNKALPAPIEKTISKAKGQPREPQVYTLPPSREEMTKN |
| | | | QVSLTCLVKGFYPSDIAVEWESNGQPENNYKTTPPVLDSDGSFFLYSKL TVDKSRWQQGNVFSCSVMHEALHNHYTQKSLSLSPGK* |
| | | QD3294 | MDMRVPAQLLGLLLLWFPGSRCDIQMTQSPSSLSASVGDRVTITCRAS QDVNTAVAWYQQKPGKAPKLLIYSASFLYSGVPSRFSGSRSGTDFTLTIS SLQPEDFATYYCQQHYTTPPTFGQGTKVEIKRTVAAPSVFIFPPSDEQLK SGTASVVCLLNNFYPREAKVQWKVDNALQSGNSQESVTEQDSKDSTY SLSSTLTLSKADYEKHKVYACEVTHQGLSSPVTKSFNRGEC* |
| Pertuzumab | QP37253726 | QD3725 | MEFGLSWLFLVAILKGVQCEVQLVESGGGLVQPGGSLRLSCAASGFTF TDYTMDWVRQAPGKGLEWVADVNPNSGGSIYNQRFKGRFTLSVDRS KNTLYLQMNSLRAEDTAVYYCARNLGPSFYFDYWGQGTLVTVSSAST KGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPK SCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK* |
| | | QD3726 | MDMRVPAQLLGLLLLWFPGSRCDIQMTQSPSSLSASVGDRVTITCKAS QDVSIGVAWYQQKPGKAPKLLIYSASYRYTGVPSRFSGSGSGTDFTLTI SSLQPEDFATYYCQQYYIYPYTFGQGTKVEIKRTVAAPSVFIFPPSDEQL KSGTASVVCLLNNFYPREAKVQWKVDNALQSGNSQESVTEQDSKDST YSLSSTLTLSKADYEKHKVYACEVTHQGLSSPVTKSFNRGEC* |
| Elotuzumab | QP34483449 | QD3448 | MEFGLSWLFLVAILKGVQCEVQLVESGGGLVQPGGSLRLSCAASGFDF SRYWMSWVRQAPGKGLEWIGEINPDSSTINYAPSLKDKFIISRDNAKNS LYLQMNSLRAEDTAVYYCARPDGNYWYFDVWGQGTLVTVSSASTKG PSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSC DKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLN GKEYKCKVSNKALPAPIEKTISKAKGQPREPQVYTLPPSRDELTKNQVS LTCLVKGFYPSDIAVEWESNGQPENNYKTTPPVLDSDGSFFLYSKLTVD KSRWQQGNVFSCSVMHEALHNHYTQKSLSLSPGK* |
| | | QD3449 | MDMRVPAQLLGLLLLWFPGSRCDIQMTQSPSSLSASVGDRVTITCKAS QDVGIAVAWYQQKPGKVPKLLIYWASTRHTGVPDRFSGSGSGTDFTLTI SSLQPEDVATYYCQQYSSYPYTFGQGTKVEIKRTVAAPSVFIFPPSDEQL KSGTASVVCLLNNFYPREAKVQWKVDNALQSGNSQESVTEQDSKDST YSLSSTLTLSKADYEKHKVYACEVTHQGLSSPVTKSFNRGEC* |
| Daratumumab | QP34503451 | QD3450 | MEFGLSWLFLVAILKGVQCEVQLLESGGGLVQPGGSLRLSCAVSGFTFN SFAMSWVRQAPGKGLEWVSAISGSGGGTYYADSVKGRFTISRDNSKN TLYLQMNSLRAEDTAVYFCAKDKILWFGEPVFDYWGQGTLVTVSSAST KGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPK SCDKTHTCPPCPAPELLGGPSVFLFPPKPKDTLMISRTPEVTCVVVDVS HEDPEVKFNWYVDGVEVHNAKTKPREEQYNSTYRVVSVLTVLHQDW LNGKEYKCKVSNKALPAPIEKTISKAKGQPREPQVYTLPPSRDELTKNQ VSLTCLVKGFYPSDIAVEWESNGQPENNYKTTPPVLDSDGSFFLYSKLT VDKSRWQQGNVFSCSVMHEALHNHYTQKSLSLSPGK* |
| | | QD3451 | MDMRVPAQLLGLLLLWFPGSRCEIVLTQSPATLSLSPGERATLSCRASQS VSSYLAWYQQKPGQAPRLLIYDASNRATGIPARFSGSGSGTDFTLTISSL EPEDFAVYYCQQRSNWPPTFGQGTKVEIKRTVAAPSVFIFPPSDEQLKS GTASVVCLLNNFYPREAKVQWKVDNALQSGNSQESVTEQDSKDSTYS LSSTLTLSKADYEKHKVYACEVTHQGLSSPVTKSFNRGEC* |
| IL15/IL15Ra-Fc | QP33123313 | QD3312 | MEFGLSWLFLVAILKGVQCNWVNVISDLKKIEDLIQSMHIDATLYTESD VHPSCKVTAMKCFLLELQVISCESGDASIHDTVENLIILANNSLSSNGN VTESGCKECEELEEKNIKEFLQSFVHIVQMFINTS* |
| | | QD3313 | MEFGLSWLFLVAILKGVQCITCPPPMSVEHADIWVKSYSLYSRERYICN SGFKRKAGTCSLTECVLNKATNVAHWTTPSLKCIRDPALVHQRSGGSG GGGSGGGSGGGGSLQEPKSSDKTHTCPPCPAPELLGGPSVFLFPPKPKD TLMISRTPEVTCVVVDVSHEDPEVKFNWYVDGVEVHNAKTKPREEQY NSTYRVVSVLTVLHQDWLNGKEYKCKVSNKALPAPIEKTISKAKGQPR EPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKS LSLSPGK* |

### Transient expression of target molecules

Expi CHO-S cells were inoculated into Forti CHO medium (Gibco, A1148301) and 8mM GlutaMax was added, then cultured at 37°C, 120rpm, 8% CO₂. The day before transfection, the cell density of Expi CHO-S was adjusted to 3*10E6/mL, placed in a shaking table, and cultured at 37°C, 120rpm and 8% CO₂. On the day of transfection, cells were taken and counted, the cell density was diluted to 6*10E6/ml, 40ml per bottle, and placed in a 125ml shake flask. 20ug of the corresponding plasmids was taken and mixed with 4.8mL Opti MEM, and 120ul Polyplus-Fecto PRO transfection reagent was added. The DNA and the transfection reagent were mixed evenly, then it was placed at room temperature for 10min. The mixture was slowly added into the cells, mixed evenly, and placed in a shaker for culture. During the culture, 2mL Feed PFF05 (OPM,F81279-001) and 1m 30% glucose solution were supplemented to each bottle on the 1st, 4th, 6th and 8th days respectively. On the first day of transfection, the temperature was reduced to 32°C and the CO₂ concentration was reduced to 5%. Samples were collected on the 13th day, centrifuged at 8000rpm for 20min, and supernatant was taken for purification.

### Example 2: Purification of fusion proteins

### Protein A Affinity Chromatography Purification

Use the equilibrium solution to pass through the column, at least 3CV, and the actual volume is 20ml. Ensure that the pH and conductivity of the final solution flowing out of the instrument were consistent with the equilibrium solution, and the flow rate was 1 ml/min; the culture supernatant after centrifugation was allowed to pass through the column, 40 ml of sample was loaded, and the flow rate was 0.33 ml/min; at least 3CV (actual volume is 20 ml) of equilibrium solution was allowed to pass through the column, so as to ensure that the pH and conductivity of the final solution flowing out of the instrument were consistent with the equilibrium solution, and the flow rate was 0.33 ml/min; the eluent was allowed to pass through the column, and the elution peaks (PAC-EP) began to be collected when the UV280 increased to 15 mAU; and collection stopped when the UV280 decreased to 15 mAU, and the flow rate was 1 ml/min. After the sample collection was collected, the PAC-EP was adjusted to neutral with a pH adjustment solution.

### CH1-XL affinity chromatography

The sample treated with Protein A was centrifuged at 8000rpm for 15 min, and the supernatant was collected. At least 3CV (actual volume 20 ml) of equilibrium solution was allowed to pass through the column, so as to ensure that the pH and conductivity of the final solution flowing out of the instrument were consistent with the equilibrium solution, and the flow rate was 1 ml/min; the supernatant after centrifugation was allowed to pass through the column through the sample loading loop, and the flow rate was 0.33 ml/min; at least 3CV (actual volume is 20 ml) of equilibrium solution was allowed to pass through the column, so as to ensure that the pH and conductivity of the final solution flowing out of the instrument were consistent with the equilibrium solution, and the flow rate was 0.33 ml/min; the eluent was allowed to pass through the column, and the elution peaks (PAC-EP) began to be collected when the UV280 increased to 10 mAU; and collection stopped when the UV280 decreased to 10 mAU, and the flow rate was 1 ml/min. After the sample collection was completed, the CH1-EP was adjusted to neutral with a pH adjustment solution.

### PNGase F enzyme digestion

39.5 µl sample was taken, 1.98 µl 10% SDS and 1.58 µl 1M DTT were added, mixed evenly and followed by boiling at 100°C for 10 minutes; when the sample was cooled, 4.8 µl 10% NP-40 and 1.0 µl PNGase F were added, mixed evenly and subjected to a water bath at 4°C overnight; Then the sample was heated at 75°C for 10 minutes to inactivate it. The sample was subjected to SDS-PAGE electrophoresis and SEC to detect protein purity.

### SDS-PAGE Electrophoresis Analysis and HPLC-SEC Analysis

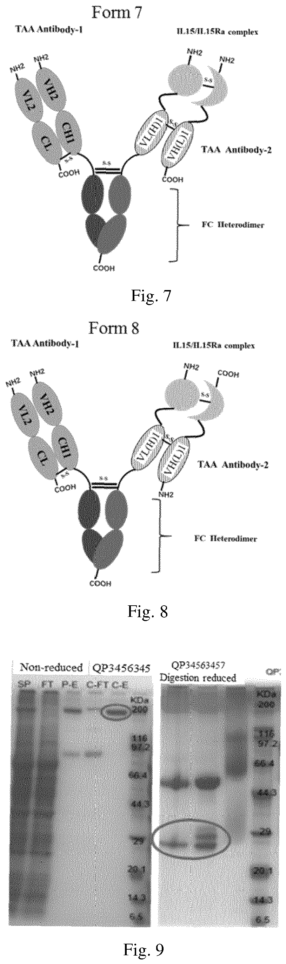

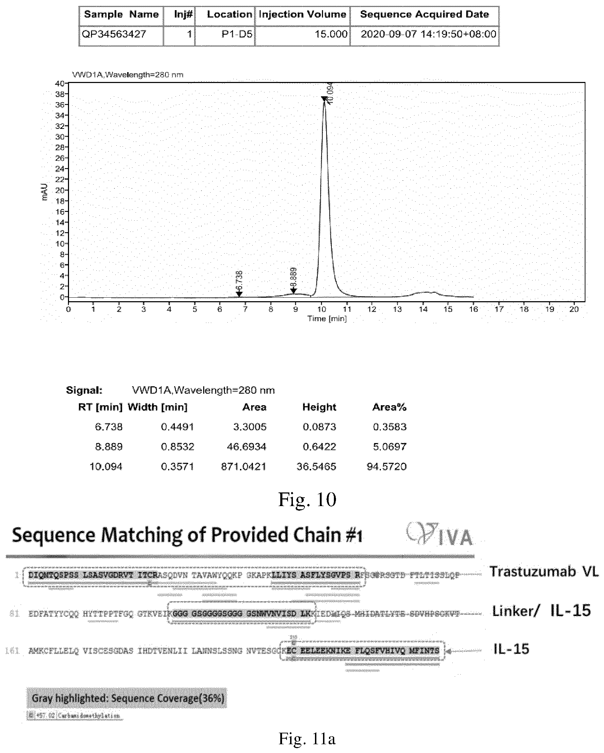

5 µg of samples before and after enzyme digestion was taken, dilute the volume to 48 µl with 1 × PBS, add 12 µl 5 × Protein Loading Dye, and heated at 95°C for 10 minutes. Tris-HCl buffer with pH 8.3 (including 0.1% SDS) was poured into the electrophoresis tank, samples were added to the sample tank with a pipette gun at 60 µl per sample tank. The voltage was set to 140 V and the time was set to 100 minutes to start electrophoresis. After electrophoresis, the gel was taken out, and soaked in the staining solution for 30 minutes, then decolorized with decolorization solution until the protein band was clear. Fig. 9 is the SDS-PAGE electrophoresis analysis of QP34563457 protein reducing and non-reducing bands. Fig. 10 is the HPLC-SEC analysis of QP34563457 protein purity.

### Example 3: Peptide Map Analysis of Protein QP34563457

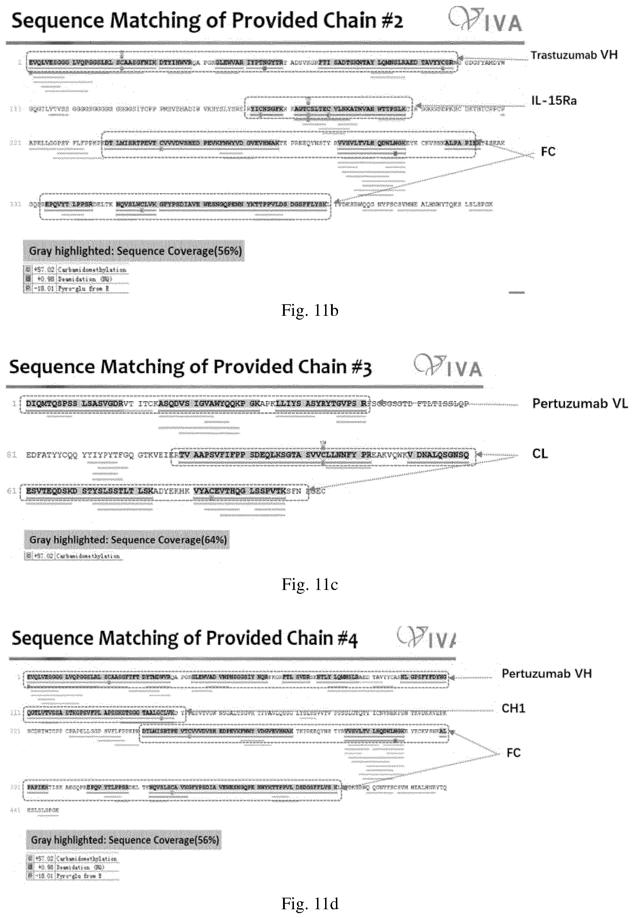

In this example, LC-MS was used to identify the amino acid sequence of QP34563457 protein. After denaturation and reduction, QP34563457 protein was added with trypsin for enzymatic hydrolysis and analyzed by LC-MS/MS (LC instrument: Agilent 1290 Infinity II, column: Agilent Peptide Plus column. MS instrument: Agilent 6545 Q-TOF). The obtained data were searched by Peaks, and the results of Peaks search were filtered by strict value restriction to obtain credible peptide segments, which achieved 36% sequence coverage of QP34563457 protein for Trastuzumab VL-IL15 chain (Fig. 11a, gray for identified amino acid sequence) and 56% sequence coverage for Trastuzumab VH-IL15Ra-Fc chain (Fig. 11b, gray for identified amino acid sequence), 64% sequence coverage for pertuzumab VL-CL chain (Fig. 11c, gray for identified amino acid sequence), and 56% sequence coverage for pertuzumab VH-CH1-Fc chain (Fig. 11d, gray for identified amino acid sequence). Through LC-MS analysis of peptide maps, various fragments in QP34563457 molecules were found, including Trastuzumab VL, IL15, Trastuzumab VH, IL15Ra, Fc, pertuzumab VL, CL, pertuzumab VH, CH1, Fc, etc.

### Example 4: ELISA Detection of the Activity of QP34563457 Binding to Her2 and Its Mutants

Design of Her2 variant protein that only binds to the Pertuzumab. Matthew C. Franklin published the complex structure of Pertuzumab Fab and Her2 extracellular structure on *Cancer cell.* The team also used alanine scanning to study which key amino acids of Her2 would affect the binding to Pertuzumab Fab. The Her2 variant protein that only binds to Pertuzumab but not trastuzumab is named Her2M2 (protein number QP3732). The amino acid sequences of Her2 wt (protein number QP3731) and Her2M2 (protein number QP3732) are as follows:
QP3731: (Her2 ECD-Fc) 
MEFGLSWLFLVAILKGVQCTQVCTGTDMKLRLPASPETHLDMLRHLYQGCQVVQ
GNLELTYLPTNASLSFLQDIQEVQGYVLIAHNQVRQVPLQRLRIVRGTQLFEDNYA
 
LAVLDNGDPLNNTTPVTGASPGGLRELQLRSLTEILKGGVLIQRNPQLCYQDTILW
KDIFHKNNQLALTLIDTNRSRACHPCSPMCKGSRCWGESSEDCQSLTRTVCAGGC
ARCKGPLPTDCCHEQCAAGCTGPKHSDCLACLHFNHSGICELHCPALVTYNTDTF
ESMPNPEGRYTFGASCVTACPYNYLSTDVGSCTLVCPLHNQEVTAEDGTQRCEKC
SKPCARVCYGLGMEHLREVRAVTSANIQEFAGCKKIFGSLAFLPESFDGDPASNTA
PLQPEQLQVFETLEEITGYLYISAWPDSLPDLSVFQNLQVIRGRILHNGAYSLTLQG
LGISWLGLRSLRELGSGLALIHHNTHLCFVHTVPWDQLFRNPHQALLHTANRPED
ECVGEGLACHQLCARGHCWGPGPTQCVNCSQFLRGQECVEECRVLQGLPREYV
NARHCLPCHPECQPQNGSVTCFGPEADQCVACAHYKDPPFCVARCPSGVKPDLSY
MPIWKFPDEEGACQPCPINCTHSCVDLDDKGCPAEQRASPLTEPKSSDKTHTCPPC
PAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK*
QP3732 (Her2M2-Fc):

MEFGLSWLFLVAILKGVQCTQVCTGTDMKLRLPASPETHLDMLRHLYQGCQVVQ
GNLELTYLPTNASLSFLQDIQEVQGYVLIAHNQVRQVPLQRLRIVRGTQLFEDNYA
LAVLDNGDPLNNTTPVTGASPGGLRELQLRSLTEILKGGVLIQRNPQLCYQDTILW
KDIFHKNNQLALTLIDTNRSRACHPCSPMCKGSRCWGESSEDCQSLTRTVCAGGC
ARCKGPLPTDCCHEQCAAGCTGPKHSDCLACLHFNHSGICELHCPALVTYNTDTF
ESMPNPEGRYTFGASCVTACPYNYLSTDVGSCTLVCPLHNQEVTAEDGTQRCEKC
SKPCARVCYGLGMEHLREVRAVTSANIQEFAGCKKIFGSLAFLPESFDGDPASNTA
PLQPEQLQVFETLEEITGYLYISAWPDSLPDLSVFQNLQVIRGRILHNGAYSLTLQG
LGISWLGLRSLRELGSGLALIHHNTHLCFVHTVPWDQLFRNPHQALLHTANRPED
ECVGEGLACHQLCARGHCWGPGPTQCVNCSQFLRGQECVEECRVLQGLPREYV
NARHCLPCHPECQPQNGSVTCFGPAADQCVACAHYKDPAFcVARCPSGVKPDLSY
MPIWKFPDEEGACQPCPINCTHSCVDLDDKGCPAEQRASPLTEPKSSDKTHTCPPC
PAPELLGGPSVFLFPPKPKDTLMISRTPEVTCVVVDVSHEDPEVKFNWYVDGVEV
HNAKTKPREEQYNSTYRVVSVLTVLHQDWLNGKEYKCKVSNKALPAPIEKTISK
AKGQPREPQVYTLPPSRDELTKNQVSLTCLVKGFYPSDIAVEWESNGQPENNYKT
TPPVLDSDGSFFLYSKLTVDKSRWQQGNVFSCSVMHEALHNHYTQKSLSLSPGK*

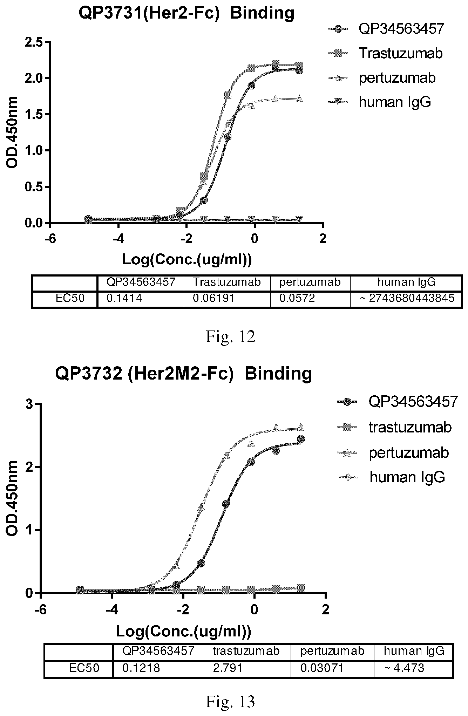

**Experimental materials:** milk (BD, 232100), PBS (Sangon, B548117-0500); HRP-anti human IgG (H + L) (jackson, 109-035-035); TMB (Luoyang Baiaotong Experimental Materials Center, C060201); Elisa plate (costa, 9018); 1 xPBS buffer: NaCl 8.00g, KCl 0.20g, Na2HPO4 • 12H2O 2.9g, KH2PO4 0.2g were weighed and added to 800mL ddH2O for dissolution, and after dissolving thoroughly, the volume was determined to 1L, and the pH was adjusted to 7.4, and followed by sterilizing at high temperature for later use. Alternatively, commercial 10× or 20× PBS solution was diluted to 1× PBS buffer for use. Blocking solution: 5g milk was weighed and added into PBS. The blocking solution shall be prepared for current use. Stop solution (1mol/L H2SO4): 109mL 98% concentrated H2SO4 was taken and slowly added dropwise into 2000mL ddH2O. After developing with TMB at 37°C for 10min, the plate was placed in a shaker (120rpm), 100 µl/well;
**Experimental process:** The plate was coated with Her2 and its variant proteins QP3731 and QP3732 at 1µg/ml, respectively, 4°C overnight, and washed with PBS for 3 times. 5% milk of blocking solution was added at 200 µL/well and incubated at 37°C for 1h. After blocking, the plate was washed with PBS for 3 times. Incubation of sample: the sample was diluted by 5 times at 20ug/ml, with a total of 7 gradients and the final well was diluted by 100 times, 100 µl/well, mixed evenly, and incubated for 1h. Then the plate was washed with PBST for 3 times. Adding enzyme labeled antibody: incubating HRP-anti human Fab antibody at a dilution ratio of 1:5000, 100 µl/well, mixed evenly, and incubated for 1h, then the plate was washed with PBST for 6 times. Adding substrate chromogenic solution: substrate chromogenic solution TMB was added at 100 µL/well. The plate was placed in a shaker, 200rpm, developing in the dark at 35°C for 10min. Termination: After the development was completed, the stop solution was immediately added at 100 µL/well to terminate the reaction. Detection: The OD value at A450nm was measured on the microplate reader, and the results were analyzed by Graphpad prism software.
**Experimental results:** It is shown that the trastuzumab can well bind to Her2 wt, and substantially does not bind Her2M2; QP34563457 and Pertuzumab can well bind to Her2 wt and Her2M2 (the results are shown in Fig. 12, Fig. 13).

### Example 5: ELISA detection of the Activity of QP34623463 binding to CD38 and CS1

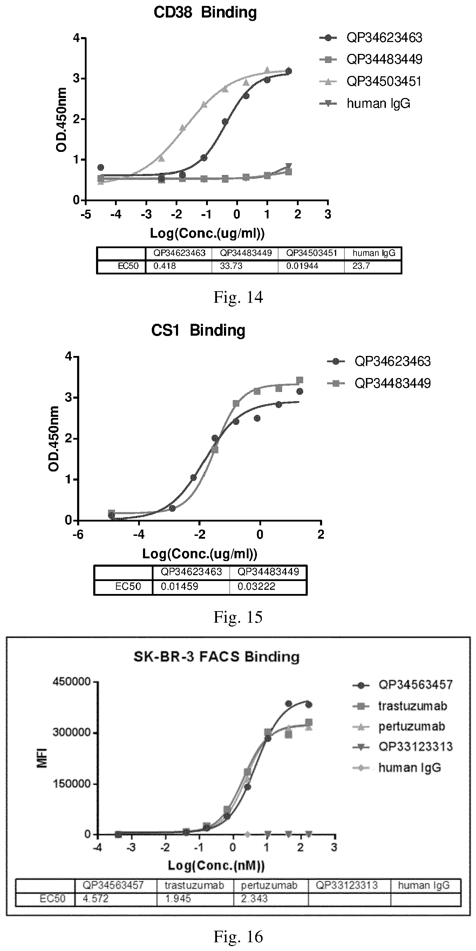

**Experimental materials:** milk (BD, 232100), PBS (Sangon, B548117-0500); HRP-anti human IgG (H + L) (jackson, 109-035-035); TMB (Luoyang Baiaotong Experimental Materials Center, C060201); Elisa plate (costa, 9018); 1 ×PBS buffer: NaCl 8.00g, KCl 0.20g, Na2HPO4 • 12H2O 2.9g, KH2PO4 0.2g were weighed and added to 800mL ddH2O for dissolution, after dissolving thoroughly, the volume was determined to 1L, and the pH was adjusted to 7.4, and followed by sterilizing at high temperature for later use. Alternatively, commercial 10× or 20× PBS solution was diluted to 1× PBS buffer for use. Blocking solution: 5g milk was weighed and added into PBS. The blocking solution shall be prepared for current use. Stop solution (1mol/L H2SO4): 109mL 98% concentrated H2SO4 was slowly added dropwise into 2000mL ddH2O. After developing with TMB at 37°C for 10min, the plate was placed in a shaker (120rpm), 100 µl/well;
**Experimental process:** The plate was coated with CD38-Fc and CS 1-Fc, respectively, at 1ug/ml, 4°C overnight, and washed with PBS for 3 times. 5% milk of blocking solution was added at 200 µL/well and incubated at 37°C for 1h. After blocking, the plate was washed with PBS for 3 times. Incubation of sample: the sample was diluted by 5 times at 20ug/ml, with a total of 7 gradients and the final well was diluted by 100 times, 100 µl/well, mixed evenly, and incubated for 1h. Then the plate was washed with PBST for 3 times. Adding enzyme labeled antibody: incubating HRP-anti human Fab antibody at a dilution ratio of 1:5000, 100 µl/well, mixed evenly, and incubated for 1h, then the plate was washed with PBST for 6 times. Adding substrate chromogenic solution: substrate chromogenic solution TMB was added at 100 µL/well. The plate was placed in a shaker, 200rpm, developing in the dark at 35°C for 10min. Termination: After the development was completed, the stop solution was immediately added at 100 µL/well to terminate the reaction. Detection: The OD value at A450nm was measured on the microplate reader, and the results were analyzed by Graphpad prism software.
**Experimental results:** QP34623463 can bind to both CD38 antigen and CS1 antigen (as shown in Fig. 14 and Fig. 15).

### Example 6: FACS detection of QP34563457 binding to Her2 over expressing cells

**Experimental materials:** BT474 cells (human breast cancer cell line), purchased from the Cell Resource Center, Institute of Basic Medical Sciences, Chinese Academy of Medical Sciences. SK-BR-3 cells (human breast cancer cell line), purchased from the Cell Resource Center, Institute of Basic Medical Sciences, Chinese Academy of Medical Sciences. SK-OV-3 cells (human ovarian cancer cell line), purchased from the Cell Resource Center, Institute of Basic Medical Sciences, Chinese Academy of Medical Sciences.

### Experimental methods:

**Experimental process:** RPMI1640 medium (Gibco) containing 10% FBS, 0.11g/L sodium pyruvate and 2.5g/L glucose was used for BT474 cells culture. DMEM medium (Gibco) containing 10% FBS was used for SK-BR-3 cells culture. McCoy's 5a medium (Gibco) containing 10% FBS was used for SK-OV-3 cells culture. Cells were cultured in a 5% CO2 incubator at 37°C.After trypsin digestion, cells were collected and inoculated into 96-well plates at 100,000 cells per well. Then the plate was subjected to blocking on ice for 1 hour with 2% FBS/PBS. The cells were incubated with different concentrations of protein QP34563457 and control protein on ice for 1 hour, washed with PBS for 3 times, and incubated with PE-anti human Fc (1:200 dilution). The cells were washed with PBS for 3 times, and resuspended with 200 ul PBS. The mean fluorescence value was read by FACS and Graphpad prism software was used to analyze the results.

**Experimental results:** QP34563457 can well bind to Her2 over expressing cells such as BT474, SK-BR-3 and SK-OV-3 cells, and its binding ability is close to that of positive control monoclonal antibodies Trastuzumab and pertuzumab, while the negative control antibody cannot bind to Her2 overexpressing cells (as shown in Figs. 16-18).

### Example 7: Her2 bispecific antibody-mediated proliferation inhibition

**Experimental objective:** To determine the inhibitory effect of Her2 bispecific antibody on proliferation of tumor cells

**Experimental materials:** BT474 cells (human breast cancer cell line), purchased from the Cell Resource Center, Institute of Basic Medical Sciences, Chinese Academy of Medical Sciences. SK-BR-3 cells (human breast cancer cell line), purchased from the Cell Resource Center, Institute of Basic Medical Sciences, Chinese Academy of Medical Sciences. SK-OV-3 cells (human ovarian cancer cell line), purchased from the Cell Resource Center, Institute of Basic Medical Sciences, Chinese Academy of Medical Sciences. Cell proliferation and toxicity detection kit (CCK-8), purchased from Meilunbio, catalog number MA0218; Human IgG, purchased from Sigma, catalog number I4506; Other antibodies were prepared internally.

**Experimental process:** RPMI1640 medium (Gibco) containing 10% FBS, 0.11g/L sodium pyruvate and 2.5g/L glucose was used for BT474 cells culture. DMEM medium (Gibco) containing 10% FBS was used for SK-BR-3 cells culture. McCoy's 5a medium (Gibco) containing 10% FBS was used for SK-OV-3 cells culture. Cells were cultured in a 5% CO₂ incubator at 37°C. After trypsin digestion, cells were collected and resuspended with a medium containing 1% FBS after centrifugation. Then the cells were inoculated into a 96-well plate at 10,000 cells, 50 µl per well, and the cells were adherent cultured at 37°C for 3 hours. Each antibody to be tested was diluted with a 3-fold gradient, evenly mixed with cell suspension at 50 µl per well, and cultured in a 37°C, 5%CO₂ incubator for 3 days. The CCK-8 reagent was added to the 96-well plate to be tested at 10 µl per well, and the plate was incubated in a 37°C, 5% CO₂ incubator for 2 hours. The 96-well plate was taken out to detect the absorbance value at 450nm wavelength in the microplate reader. The cell viability value was calculated, and plotted with the logarithm of sample concentration, and the results were analyzed by Graphpad prism software. The cell proliferation inhibition curve was fitted by four parameters.

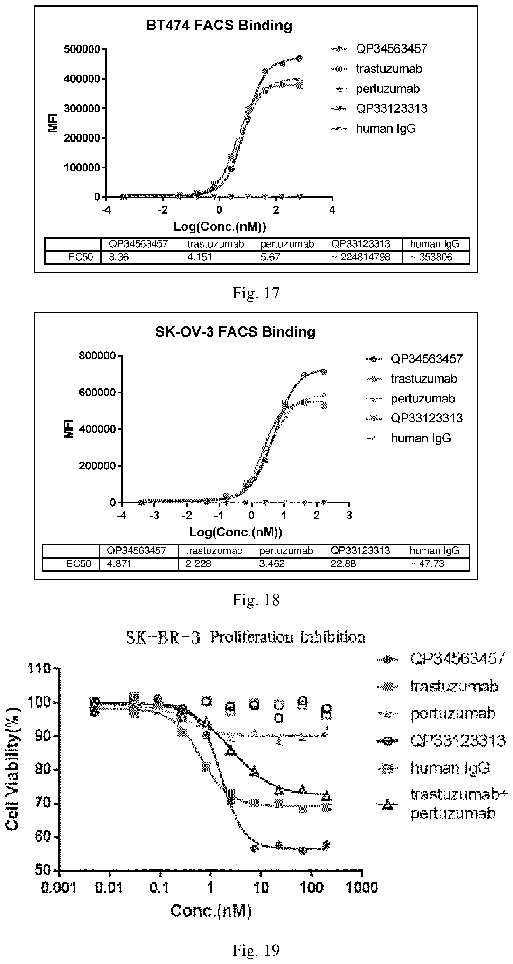

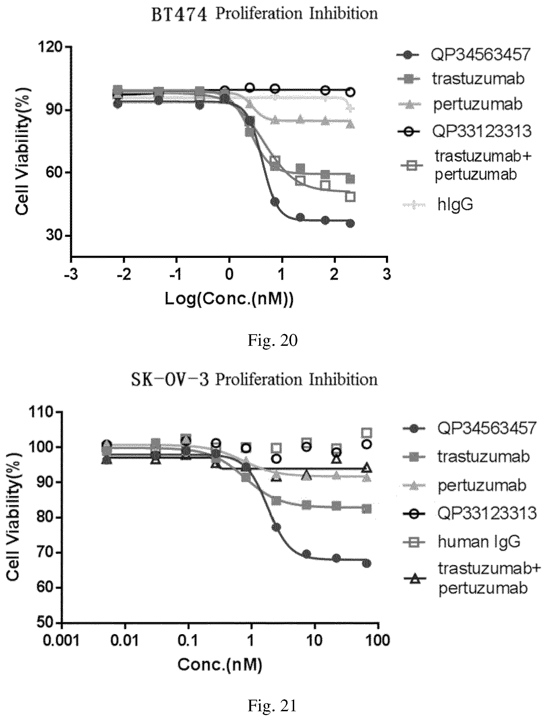

**Experimental results:** QP34563457 shows significant proliferation inhibitory effect on HER2-positive tumor cells BT474, SK-BR-3 and SK-OV-3, which is significantly better than Trastuzumab or Pertuzumab and the combination of both (with synergistic effect) (as shown in Figs. 19-21).

### Example 8: HER2 bispecific antibody-mediated ADCC effect

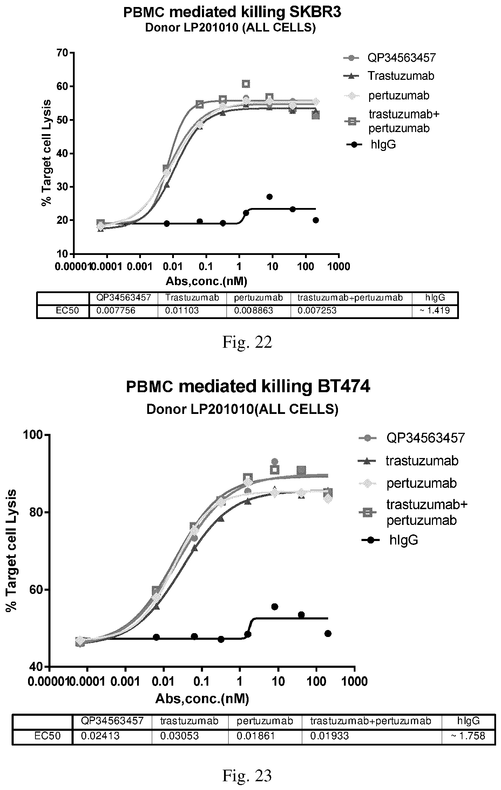

**Experimental objective:** To determine the ADCC effect of HER2 bispecific antibody
**Experimental materials:** PBMC, purchased from Shanghai Saili Biotechnology Co., Ltd. BT474 cells (human breast cancer cell line), purchased from the Cell Resource Center of Institute of Basic Medicine, Chinese Academy of Medical Sciences. SK-BR-3 cells (human breast cancer cell line), purchased from the Cell Resource Center, Institute of Basic Medical Sciences, Chinese Academy of Medical Sciences. SK-OV-3 cells (human ovarian cancer cell line), purchased from the Cell Resource Center of Institute of Basic Medicine, Chinese Academy of Medical Sciences. Cytotox96 non-radioactive cytotoxicity assay test kit, purchased from Promega (G1780).
**Experimental process:** PBMCs were resuscitated and cells were collected for later use the next day. Preparation of target cells: SK-BR-3, BT-474, SK-OV-3 were digested with trypsin, 1000rpm 5min. After washed with PBS twice, the cells were inoculated into 96-well plates at 20,000 cells per well, 50 µl per well, and incubated at 5% CO₂, 37°C for 2 hours. Preparation of antibody: The antibody was diluted with a gradient of 1:4 (80ug/ml - 0.000512ug/ml, 0ug/ml) using culture medium (RPMI1640 containing 10% low IgG FBS) for 10 concentrations. The above diluted antibody in each concentration was added at 50 µl per well and incubated at 37°C for 15min. Preparation of PBMC: PBMCs resuscitated on the first day were centrifuged, resuspended in culture medium (RPMI1640 containing 10% low IgG FBS), and were counted. According to PBMC: Target cell = 30:1, it was added into the well-plate at 50 µl per well and incubated at 37°C for 4 hours. LDH detection: for the maximum release group and volume correction group, 10 µl lysate was added 45min in advance, and continued to be cultured in the incubator. After culture for 4h, 50 µl of supernatant was absorbed into the enzyme label plate, and 50 µl of reagent was added according to the instructions of Cytotox96 non-radioactive cytotoxicity assay kit (Promega, G1780). After reaction in the dark at room temperature for 30 min, 50 µl stop solution was added, and absorbance value at 490nm was read (the reading was completed within 1h after adding stop solution). Calculation: killing percentage = (sample release - target cell spontaneous release - effector cell spontaneous release)/(target cell maximum release - target cell spontaneous release) * 100. Spontaneous release (corresponding to target cells incubated with effector cells in the absence of antibodies) was defined as 0% cytotoxicity, and maximum release (target cells lysed with 1% Triton X-100) was defined as 100% cytotoxicity.
**Experimental results:** It is indicated that QP34563457 maintains ADCC activity comparable to the two HER2 monoclonal antibodies in BT474, SK-BR-3, and SK-OV-3 tumor cells (as shown in Figs. 22-24).

### Example 9: Mo7e cell proliferation experiment

**Experimental materials:** Mo7e cells (human giant cell leukemia cell line), purchased from the Cell Resource Center of Institute of Basic Medicine, Chinese Academy of Medical Sciences. Cell Proliferation and Toxicity Test Kit (CCK-8), purchased from Meilunbio, catalog number MA0218; Recombinant human GM-CSF, purchased from Perprotech, catalog number 300-03; Human IgG, purchased from Sigma, catalog number I4506.Other antibodies were prepared internally.

**Experimental process:** Mo7e cells were cultured in RPMI1640 medium containing 10% FBS, 2mM L-glutamine and 8ng/m1 GM-CSF in a 5% CO₂ incubator at 37°C. Mo7e cells were collected, centrifuged at 800rpm for 5 minutes to pour out the supernatant, and the cells were washed twice with RPMI1640 medium without GM-CSF. The cells were resuspended with GM-CSF-free RPMI1640 medium and counted, then inoculated into 96-well plates at 2 × 10⁴ cells with80 µl per well, and cultured in a 5% CO₂ incubator at 37°C for 1 hour. Each antibody to be tested was diluted with a 4-fold gradient in the culture medium, evenly mixed with cell suspension at 20 µl per well, and the plate was cultured in a 5% CO₂incubator at 37°C for 3 days. The CCK-8 reagent was added to the 96-well plate to be tested at 10 µl per well, and incubated in a 5% CO₂ incubator at 37°C for 4 hours. The 96-well plate was taken out and the absorbance value at 450nm wavelength was detected in the microplate reader.

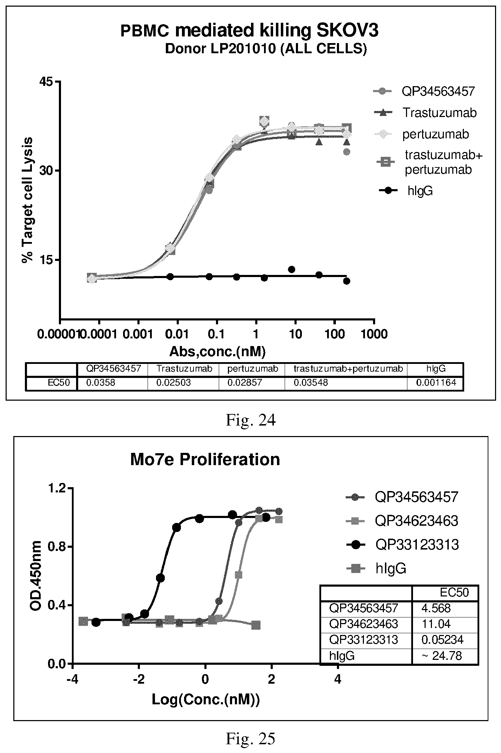

**Experimental results:** The proliferation experiment of Mo7e cells verified that it has appropriate IL15 activity (as shown in Fig. 25).

### Example 10: Animal Pharmacodynamic Assay

The inhibitory effect of QP34563457 was evaluated on tumor growth in a human ovarian cancer cell SK-OV-3 model with high HER2 expression.

**Experimental process:** The SK-OV-3 cells were cultured in McCoy's 5A + 10% FBS culture medium containing 10% fetal bovine serum. SK-OV-3 cells in the exponential growth phase were collected and resuspended with PBS to a suitable concentration for inoculation. Each BALB/C-nude mouse was subcutaneously inoculated with 1 × 10⁶ SK-OV-3 cells on the right back, and the tumor growth was regularly observed. When the tumor grew to an average volume of about 50mm³, the mice were randomly divided into groups according to the tumor size and the weight of the mice for administration (the dosage volume was 10ul/g body weight), and the day of the first administration was defined as D0. The administration regimen is as follows:

**Table 3: Administration regimen**

| Group | Animal Number s | Administration group | Dosage (mg/kg ) | Administratio n method | Administratio n cycle |
|---|---|---|---|---|---|
| 1 | 10 | PBS | -- | i.p. | Q3D × 6 |
| 2 | 10 | Tastuzumab | 5 | i.p. | Q3D × 6 |
| 3 | 10 | Pertuzumab | 5 | i.p. | Q3D × 6 |
| 4 | 10 | Trastuzumab+Pertuzuma b | 5+5 | i.p. | Q3D × 6 |
| 5 | 7 | QP34563457 | 0.5 | i.p. | Q3D × 6 |

The body weight and tumor volume of mice were observed and recorded for each administration. The tumor length diameter was measured with vernier caliper, and the tumor growth was calculated and recorded according to the Formula: tumor volume (mm³) = 0.5 × (a × b²), and the tumor growth curve was plotted. After the last administration, the observation was conducted for 6 days, and the experiment was ended and the experimental data were recorded.

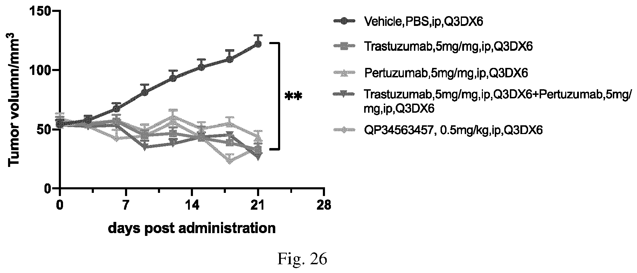

**Experimental results:** The results show that Tastuzumab, Pertuzumab, Trastuzumab + Pertuzumab and QP34563457 can significantly inhibit the growth of SK-OV-3 tumor, and the tumor regressed after 6 doses (TGItv>100%), proving that QP34563457 still has significant tumor growth inhibitory activity in huPBMC-graft NCG mouse model under the premise of lower dosage (10% of monoclonal antibody dosage) (as shown in Fig. 26 and Table 4).

**Table 4: SK-OV-3 in vivo pharmacodynamic data and analysis**

| **Group** | **D0 Tumor volume mm³** | **D21 Tumor volume mm³** | **D21 Percentage of tumor growth inhibition** | **P value *v*.*s*. vehicle** |
|---|---|---|---|---|
| Vehicle, PBS | 54.07±3.72/D0 | 121.94±7.43 /D21 | -- | -- |
| Tastuzumab, 5mg/kg | 55.32±4.99/D0 | 32.7±5.07 /D21 | 133.33 | <0.0001** |
| Pertuzumab, 5mg/kg | 53.58+4.25/D0 | 43.72±5.02 /D21 | 114.56 | <0.0001** |
| Tastuzumab, 5mg/kg + Pertuzumab, 5mg/kg | 53.81±3.66/D0 | 26.62±3.39 /D21 | 140.06 | <0.0001** |
| QP34563457, 0.5mg/kg | 54.57±4.17/D0 | 34.98±5.29 /D21 | 121.79 | <0.0001** |

Although specific embodiments of the present invention have been described in detail, it will be understood by those skilled in the art that according to all the teachings disclosed, various modifications and substitutions can be made to those details, which are all within the protection scope of the present invention. The full scope of the present invention is given by the appended claims and any equivalents thereof.

## Claims

1. A bispecific antibody, which comprises:
a) a light chain and a heavy chain of a first antibody that specifically binds to a first antigen; and
b) a light chain and a heavy chain of a second antibody that specifically binds to a second antigen;
wherein the two chains of the first antibody comprise IL15 and IL15Rα, respectively, and are capable of forming an IL15/IL15Rα complex;
the IL15 comprises a mutant capable of binding to IL15Rα, and the IL15Rα comprises a mutant capable of binding to IL15.

2. The bispecific antibody of claim 1, there are one or more pairs of disulfide bonds between the IL15 and IL15Rα
and the IL15 comprises the following mutations, the counting method is that the first amino acid of IL15 as shown in SEQ ID No.1 is counted as the 1st position;
| Combination number | **IL15 mutation** |
|---|---|
| 1 | N1D |
| 2 | N4D |
| 3 | D8N |
| 4 | D30N |
| 5 | D61N |
| 6 | E64Q |
| 7 | N65D |
| 8 | Q108E |
| 9 | N1D/D61N |
| 10 | N1D/E64Q |
| 11 | N4D/D61N |
| 12 | N4D/E64Q |
| 13 | D8N/D61N |
| 14 | D8N/E64Q |
| 15 | D61N/E64Q |
| 16 | E64Q/Q108E |
| 17 | N1D/N4D/D8N |
| 18 | D61N/E64Q/N65D |
| 19 | N1D/D61N/E64Q/Q108E |
| 20 | N4D/D61N/E64Q/Q108E |
or the IL15 and IL15Rαcomprise the following mutation combinations, and the counting method is that the first amino acid of IL15 as shown in SEQ ID No.1 is counted as the 1st position; the first amino acid of IL15Rα as shown in SEQ ID No.3 is counted as the 1st position;
| **Combination number** | **IL15** | **IL15Ra** |
|---|---|---|
| 1 | wt | D96 |
| 2 | wt | D96/P97 |
| 3 | wt | D96/P97/A98 |
| 4 | E87C | D96/C97 |
| 5 | E87C | D96/P97/C98 |
| 6 | E87C | D96/C97/A98 |
| 7 | V49C | S40C |
| 8 | L52C | S40C |
| 9 | E89C | K34C |
| 10 | Q48C | G38C |
| 11 | E53C | L42C |
| 12 | C42S | A37C |
| 13 | L45C | G38C |
| 14 | L45C | A37C. |

3. The bispecific antibody of claim 1, there are one or more pairs of disulfide bonds between the variable domains VH1 and VL1 of the first antibody; and the VH1 and VL1 comprise the following mutation combination forms, according to EU numbering;
| | **Modification site of disulfide bond** | |
|---|---|---|
| | VH | VL |
| Combination 1 | 37C | 95C |
| Combination 2 | 44C | 100C |
| Combination 3 | 44C | 105C |
| Combination 4 | 45C | 87C |
| Combination 5 | 100C | 50C |
| Combination 6 | 100bC | 49C |
| Combination 7 | 98C | 46C |
| Combination 8 | 101C | 46C |
| Combination 9 | 105C | 43C |
| Combination 10 | 106C | 57C. |

4. The bispecific antibody of claim 1, wherein the heavy chain of the first antibody and the second antibody comprises a Fc segment, which comprises a chain A and a chain B with different mutations, respectively, and the chain A and the chain B have the following mutation combination forms, according to EU numbering.
| Combination number | FC | Heterodimer mutation (Eu numbering) |
|---|---|---|
| 1 | FC-A chain | T366Y |
| | FC-B chain | Y407T |
| 2 | FC-A chain | T366W |
| | FC-B chain | T366S/L368A/Y407V |
| 3 | FC-A chain | S354C/T366W |
| | FC-B chain | Y349C/T366S/L368A/Y407V |
| 4 | FC-A chain | S364H/F405A |
| | FC-B chain | Y349T/T394F |
| 5 | FC-A chain | T350V/L351 Y/F405A/Y407V |
| | FC-B chain | T350V/T366L/K392L/T394W |
| 6 | FC-A chain | K392D/K409D |
| | FC-B chain | E356K/D399K |
| 7 | FC-A chain | D221 E/P228E/L368E |
| | FC-B chain | D221 R/P228R/K409R |
| 8 | FC-A chain | K360E/K409W |
| | FC-B chain | Q347R/D399V/F405T |
| 9 | FC-A chain | K360E/K409W/Y349C |
| | FC-B chain | Q347R/D399V/F405T/S354C |
| 10 | FC-A chain | K370E/K409W |
| | FC-B chain | E357N/D399V/F405T |
| 11 | FC-A chain | F405L |
| | FC-B chain | K409R |
| 12 | FC-A chain | K360D/D399M/Y407A |
| | FC-B chain | E345R/Q347R/T366V/K409V |
| 13 | FC-A chain | Y349S/K370Y/T366M/K409V |
| | FC-B chain | E356G/E357D/S364Q/Y407A |
| 14 | FC-A chain | L351 D/L368E |
| | FC-B chain | L351 K/T366K |
| 15 | FC-A chain | GQPFRPEVHLLPPSREEMTKNQVSLTCLARGFYPKDIAVEWES NGQPENNYKTTPSRQEPSQGTTTFAVTSKLTVDKSRWQQGNV FSCSVMHEALHNHYTQKTISL |
| | FC-B chain | GQPREPQVYTLPPPSEELALNELVTLTCLVKGFYPSDIAVEWLQ GSQELPREKYLTWAPVLDSDGSFFLYSILRVAAEDWKKGDTFS CSVMHEALHNHYTQKSLDR |
| 16 | FC-A chain | L368D/K370S |
| | FC-B chain | E357Q/S364K |
| 17 | FC-A chain | S354C/T366W/K409A |
| | FC-B chain | Y349C/T366S/L368A/Y407V/F405K |
| 18 | FC-A chain | S354C/T366W/F405K/K360EQ347E |
| | FC-B chain | Y349C/T366S/L368AIY 407V/034 7RIT394W |
| 19 | FC-A chain | T366W/K409A |
| | FC-B chain | T366S/L368G/Y407A/F405K |
| 20 | FC-A chain | knobs(T366W/F405K) |
| | FC-B chain | holes(T366S/L368G/Y407A/K409A) |
| 21 | FC-A chain | Q347A/S364K/T366V/K370T/K392Y/F405S/Y407V/K409W/T411 N |
| | FC-B chain | Q347E/Y349A/L351F/S364T/T366V//K370T/T394D/V397L/D399E /D401Q/F405A/Y407S/K409R/T411R |
| 22 | FC-A chain | K274Q/N276K/Y300F/A339T/Q347A/S364K/T366V/K370T/N384S /K392Y/V397M/F405S/Y407V/K409W/T411N/V422I/H435R/Y436 F |
| | FC-B chain | Q347E/Y349A/L351F/S364T/T366V//K370T/T394D/V397L/D399E /D401Q/F405A/Y407S/K409R/T411R |

5. The bispecific antibody of claim 4, wherein the Fc segment comprises Human IgG1 Fc, Human IgG2 Fc, Human IgG3 Fc, Human IgG4 Fc and mutants thereof; for the chain A and chain B of the Fc segment, wherein one chain is capable of binding to protein A and the other chain is a mutant incapable of binding to protein A, and the mutation comprises H435R or H435R/Y436F, according to EU numbering.

6. The bispecific antibody of claim 1, wherein the first antigen is any one of CD3, CD20, CD19, CD30, CD33, CD38, CD40, CD52, slamf7, GD2, CD24, CD47, CD133, CD217, CD239, CD274, CD276, PD-1, CEA, Epcam, Trop2, TAG72, MUC1, MUC16, mesothelin, folr1, CLDN18.2, PDGFR2,FVIII, C- MET, EGFR, EGFR, SCA ephA2, ADAM17, 17-A1, NKG2D ligands, MCSP, LGRS, SSEA3, SLC34A2, BCMA, GPNMB, IL-6R, IL-2R, CCR4, VEGFR-2, CD6, CTLA-4, integrin α4, DNA/histone complex, PDGFRα, NeuGcGM3, IL-4Rα, IL-6Rα, the second antigen is an epitope different from the first antigen or another antigen as described above.

7. The bispecific antibody of claim 1, wherein the first and/or second antibody is a chimeric, humanized or fully human antibody.

8. The bispecific antibody of any one of claims 1-7, which has a structure shown in Formula I or Formula II: 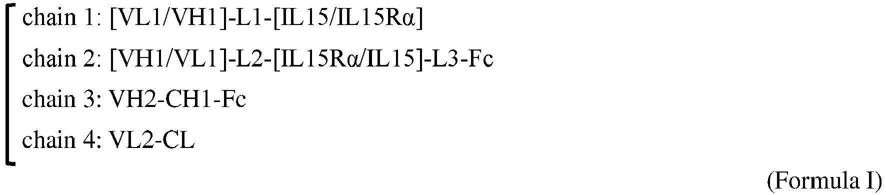 wherein,
chain 1: VL1 or VH1 is linked to the N- or C-terminus of IL15 or IL15Rα via L1;
chain 2: arranged from N-terminus to C-terminus is VH1 or VL1, L2, IL15Rα or IL15, L3, Fc;
chain 3: the heavy chain of the second antibody arranged from the N-terminus to the C-terminus is VH2-CH1-Fc;
chain 4: the light chain of the second antibody arranged from the N-terminus to the C-terminus isVL2-CL;
VH1 and VL1 represent the variable domains of the first antibody;
VH2 and VL2 represent the variable domains of the second antibody;
CH1 and CL represent the constant domain of the second antibody;
"-" represents a peptide bond;
L1, L2 and L3 are each independently a bond or a linker sequence; 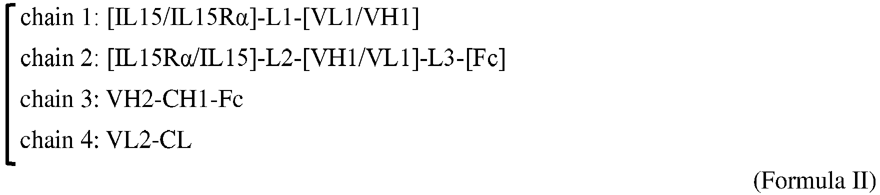 wherein,
chain 1: II,15 or IL15Rα is linked to the N- terminus or C-terminus of VL1 or VH1 via L1;
chain 2: arranged from N-terminus to C-terminus isIL15Rα or IL15, L2, VH1 or VL1, L3, Fc;
chain 3: the heavy chain of the second antibody arranged from the N-terminus to the C-terminus isVH2-CH1-Fc;
chain 4: the light chain of the second antibody arranged from the N-terminus to the C-terminus isVL2-CL;
VH1 and VL1 represent the variable domains of the first antibody;
VH2 and VL2 represent the variable domains of the second antibody;
CH1 and CL represent the constant domain of the second antibody;
"-" represents a peptide bond;
L1, L2 and L3 are each independently a bond or a linker sequence.

9. The bispecific antibody of claim 8, wherein the chain 1 and chain 2 comprise the following combination forms:
| | | |
|---|---|---|
| Combination 1 | Chain 1: | VL1-L1-IL15 |
| | Chain 2: | VH1-L2-IL15Ra-L3-Fc (chain A or B) |
| Combination 2 | Chain 1: | IL15-L1-VL1 |
| | Chain 2: | VH1-L2-IL15Ra-L3-Fc (chain A or B) |
| Combination 3 | Chain 1: | VL1-L1-IL15 |
| | Chain 2: | IL15Ra-L2-VHl-L3-Fc (chain A or B) |
| Combination 4 | Chain 1: | IL15-L1-VL1 |
| | Chain 2: | IL15Ra-L2-VHl-L3-Fc (chain A or B) |
| Combination 5 | Chain 1: | VH1-L1-IL15 |
| | Chain 2: | VL1-L2-IL15Ra-L3-Fc (chain A or B) |
| Combination 6 | Chain 1: | IL15-L1-VH1 |
| | Chain 2: | VL1-L2-IL15Ra-L3-Fc (chain A or B) |
| Combination 7 | Chain 1: | VH1-L1-IL15 |
| | Chain 2: | IL15Ra-L2-VLl-L3-Fc (chain A or B) |
| Combination 8 | Chain 1: | IL15-L1-VH1 |
| | Chain 2: | IL15Ra-L2-VLl-L3-Fc (chain A or B) |
| Combination 9 | Chain 1: | VL1-L1-IL15Ra |
| | Chain 2: | VH1-L2-IL15-L3-Fc (chain A or B) |
| Combination 10 | Chain 1: | IL15Ra-Ll-VLl |
| | Chain 2: | VH1-L2-IL15-L3-Fc (chain A or B) |
| Combination 11 | Chain 1: | VL1-L1-IL15Ra |
| | Chain 2: | IL15-L2-VH1-L3-Fc (chain A or B) |
| Combination 12 | Chain 1: | IL15Ra-Ll-VLl |
| | Chain 2: | IL15-L2-VH1-L3-Fc (chain A or B) |
| Combination 13 | Chain 1: | VH1-L1-IL15Ra |
| | Chain 2: | VL1-L2-IL15-L3-Fc (chain A or B) |
| Combination 14 | Chain 1: | IL15Ra-Ll-VHl |
| | Chain 2: | VL1-L2-IL15-L3-Fc (chain A or B) |
| Combination 15 | Chain 1: | VH1-L1-IL15Ra |
| | Chain 2: | IL15-L2-VL1-L3-Fc (chain A or B) |
| Combination 16 | Chain 1: | IL15Ra-Ll-VHl |
| | Chain 2: | IL15-L2-VL1-L3-Fc (chain A or B). |

10. The bispecific antibody of claim 8, which has a structure shown in Formula III from N-terminus to C-terminus: 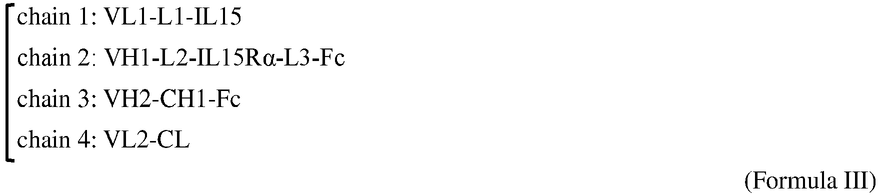 wherein,
VH1 and VL1 represent the variable domains of the first antibody;
VH2 and VL2 represent the variable domains of the second antibody;
CH1 and CL represent the constant domain of the second antibody;
"-" represents a peptide bond;
L1, L2 and L3 are each independently a low immunogenic amino acid linker sequence.

11. The bispecific antibody of any one of claims 8-10, wherein the IL15 sequence is shown in SEQ ID No.1 or SEQ ID No.2; the IL15Rα sequence is shown in SEQ ID No.3, SEQ ID No.4, SEQ ID No.5, SEQ ID No.6, SEQ ID No.7, SEQ ID No.8 or SEQ ID No.9.

12. The bispecific antibody of any one of claims 8-10, wherein the Fc sequence is shown in SEQ ID No.18 or SEQ ID No.19.

13. The bispecific antibody of claim 1, wherein the first antigen and the second antigen are antigens binding to two different epitopes of Her2, respectively.

14. The bispecific antibody of claim 12, wherein the bispecific antibody is obtained by expressing fusion sequences of SEQ ID No.10, SEQ ID No.11, SEQ ID No.13, SEQ ID No.12.

15. The bispecific antibody of claim 1, wherein the first antigen and the second antigen are CS1 antigen and CD38 antigen, respectively.

16. The bispecific antibody of claim 14, wherein the bispecific antibody is obtained by expressing fusion sequences of SEQ ID No.14, SEQ ID No.15, SEQ ID No.17, SEQ ID No.16.

17. A pharmaceutical composition comprising:
(a) the bispecific antibody of any one of claims 1-16; and
(b) a pharmaceutically acceptable carrier.

18. Use of the bispecific antibody of any one of claims 1-16 in the preparation of a medicament for treating cancer (or tumor), infectious or immunomodulatory disease.

19. Use of the bispecific antibody of any one of claims 1-16 in the preparation of a medicament for inhibiting tumor growth.

20. The bispecific antibody of claim 18 or 19, wherein the cancer (or tumor) comprises: colorectal cancer, breast cancer, ovarian cancer, pancreatic cancer, gastric cancer, prostate cancer, renal cancer, cervical cancer, thyroid cancer, endometrial cancer, uterine cancer, bladder cancer, neuroendocrine cancer, head and neck cancer, liver cancer, nasopharyngeal cancer, testicular cancer, myeloma, lymphoma, leukemia, small cell lung cancer, non-small cell lung cancer, melanoma, basal cell skin cancer, squamous cell skin cancer, carina Dermatofibrosarcoma,, Merkel cell carcinoma, glioblastoma, glioma, sarcoma, mesothelioma, and myelodysplastic syndrome.

21. Use of the bispecific antibody of claim 14 in the preparation of a reagent or a kit for diagnosing a HER2 positive tumor (such as breast cancer and gastric cancer).
